# EUROPEAN PATENT SPECIFICATION

(11) **EP 3 153 207 B1**
(45) Date of publication and mention of the grant of the patent: **18.09.2019**
(21) Application number: 16192481.6
(22) Date of filing: 05.10.2016
(51) Int. Cl.: A61K 47/02, A61K 47/36, A61K 47/38, A61K 9/70, A61K 38/38, A61M 37/00

(54) **METHOD OF MANUFACTURING TRANSDERMAL ABSORPTION SHEET**
VERFAHREN ZUR HERSTELLUNG EINES TRANSDERMALEN RESORPTIONSBLATTS
PROCÉDÉ DE FABRICATION DE CHAMP D'ABSORPTION TRANSDERMIQUE

(30) Priority: 06.10.2015 JP 2015198258
(43) Date of publication of application: 12.04.2017
(73) Proprietor: FUJIFILM Corporation, Tokyo 106-8620 (JP)
(72) Inventor: WAKAMATSU, Satoshi, Kanagawa, 258-8538 (JP)
(74) Representative: Klunker IP Patentanwälte PartG mbB

(56) References cited:
- KR-B1- 101 549 086
- US-A1- 2003 135 161
- US-A1- 2004 162 542
- US-A1- 2009 118 662

## Description

### BACKGROUND OF THE INVENTION

### Field of the Invention

The present invention relates to a method of manufacturing a transdermal absorption sheet having needle like protruding portions.

### Description of the Related Art

US 2003/135161 A1 teaches a micro needle device and methods for manufacturing the micro needle devices.

In order to deliver drugs into skins, transdermal absorption sheets each having a plurality of needle-like protruding portions (also referred to as "microneedles") containing the drugs have been recently used. In general, a drug in needle-like protruding portions is delivered into a skin by pressing a transdermal absorption sheet against the skin and inserting the needle-like protruding portions into the skin.

For such transdermal absorption sheets, various proposals have been made. For example, Japanese Patent Application Laid-Open No. 2008-006178 discloses a method of manufacturing a microneedle sheet (a transdermal absorption sheet) having a microneedle array on a surface of a resin polymer, the method including applying a solution in which a resin polymer is dissolved, to a stamper for forming the microneedle array, drying the solution, putting a sheet-like substrate on the surface of the dried polymer coagulate to be attached thereto, and releasing the sheet-like substrate and the resin polymer coagulate from the stamper.

In addition, Japanese Patent Application Laid-Open No. 2011-012050 discloses a microneedle-array base plate at least one of the surfaces of which is a flat surface, wherein protruding portions are provided on a surface opposite to the flat surface, a portion from the flat surface to the surface opposite to the flat surface is made of a porous material.

### SUMMARY OF THE INVENTION

However, the technique disclosed in Japanese Patent Application Laid-Open No. 2008-006178 may generate, in the manufacturing method, a curl due to volume contraction when the solution is dried. In addition, when the manufactured microneedle sheet is used in a high humidity environment, the sheet portion may be curled by absorbing moisture, or the sheet portion may be broken when being punctured.

In the technique disclosed in Japanese Patent Application Laid-Open No. 2011-012050, a high-molecular substance is immersed in a porous material, dried, and cured. Accordingly, the technique presents a problem in that a drying time becomes long.

The present invention has been made in view of the above circumstances, and provides a method of manufacturing a transdermal absorption sheet capable of suppressing a sheet portion from being curled and improving impact resistance of the sheet portion, capable of suppressing the sheet portion from being curled in drying, and shortening a base-material liquid drying time.

The transdermal absorption sheet includes: a sheet portion; a plurality of needle-like protruding portions that are arranged on one surface of the sheet portion; and a mesh structure body that has a sheet-like shape and is included in the sheet portion, the mesh structure body having an area, in a plan view, which covers at least a part of a region in which the plurality of needle-like protruding portions are arranged.

It is preferable that the mesh structure body is made of a metal.

It is preferable that the metal is stainless steel.

It is preferable that the mesh structure body is embedded in the sheet portion.

It is preferable that the mesh structure body includes a portion embedded in the sheet portion and a portion exposed from the sheet portion.

It is preferable that the mesh structure body is a woven net structure.

It is preferable that the number of meshes of the mesh structure body is within a range of 12 to 635 meshes, and a wire diameter of the mesh structure body is within a range of 0.02 to 1.0 mm.

It is preferable that the needle-like protruding portion includes a first layer containing a drug and a second layer not containing a drug.

It is preferable that the drug is at least one of peptide, protein, nucleic acid, polysaccharide, a vaccine, a medical compound, and a cosmetic component.

According to the present invention, a method of manufacturing a transdermal absorption sheet, as described in claim 1, includes in this order: a filling step of placing, on a mold having two-dimensionally arranged needle-like recessed portions, a mesh structure body which has an area, in a plan view, covering at least a region of the needle-like recessed portions, and filling the needle-like recessed portions with a polymer solution via the mesh structure body; a drying step of drying the polymer solution filled in the needle-like recessed portions; and a releasing step of releasing the dried polymer solution and the mesh structure body from the mold.

According to still another aspect, a method of manufacturing a transdermal absorption sheet, includes in this order: a liquid drug filling step of filling, with a liquid drug which is a polymer solution containing a drug, needle-like recessed portions of a mold in which the needle-like recessed portions are two dimensionally arranged; a liquid drug drying step of drying the liquid drug filled in the needle-like recessed portions, to form a first layer containing the drug; a base-material liquid filling step of placing a mesh structure body on the mold, the mesh structure having an area which covers at least a region of the needle-like recessed portions in a plan view, and filling the needle-recessed portions, on the first layer, via the mesh structure body with a base material liquid which is a polymer solution not containing a drug; a base-material liquid drying step of drying the base material liquid to form, on the first layer, a second layer not containing a drug; and a releasing step of releasing the first layer, the second layer, and the mesh structure body from the mold.

According to the method of manufacturing a transdermal absorption sheet of the present invention, the sheet portion can be suppressed from being curled and the impact resistance of the sheet portion can be improved. In addition, according to the method of manufacturing the transdermal absorption sheet of the present invention, the sheet portion can be suppressed from being curled in drying and a time required for drying a base material liquid can be shortened.

### BRIEF DESCRIPTION OF THE DRAWINGS

Fig. 1 is an entire perspective view of a transdermal absorption sheet;
Fig. 2 is a cross-sectional view of the transdermal absorption sheet;
Fig. 3 is a plan view of a mesh structure body;
Fig. 4 is a cross-sectional view of a transdermal absorption sheet of another embodiment;
Fig. 5 is a partially enlarged view of the transdermal absorption sheet having a needle-like protruding portion;
Fig. 6 is a partially enlarged view of the transdermal absorption sheet having a needle-like protruding portion which has another shape;
Fig. 7 is a cross-sectional view of the needle-like protruding portion of the transdermal absorption sheet illustrated in Figs. 5 and 6;
Fig. 8 is a perspective view of the transdermal absorption sheet having a needle-like protruding portion which has another shape;
Fig. 9 is a perspective view of the transdermal absorption sheet having a needle-like protruding portion which has still another shape;
Fig. 10 is a cross-sectional view of the needle-like protruding portion of the transdermal absorption sheet illustrated in Figs. 8 and 9;
Fig. 11A to 11C are diagrams of processes of a manufacturing method for a mold;
Figs. 12A and 12B are diagrams each illustrating the mold provided with a frame;
Fig. 13 is a partially enlarged view of the mold;
Fig. 14 is a partially enlarged view of a mold composite body;
Fig. 15 is a flowchart of a method of manufacturing the transdermal absorption sheet;
Figs. 16A to 16C are schematic diagrams illustrating processes for filling needle-like recessed portions of the mold with a liquid drug;
Fig. 17 is a perspective view of the tip of a nozzle;
Fig. 18 is a perspective view of the tip of another nozzle;
Fig. 19 is a partially enlarged view of the tip of the nozzle and the mold which filling is performed;
Fig. 20 is a partially enlarged view of the tip of the nozzle and the mold which scanning is performed;
Fig. 21 is a schematic configuration diagram of a liquid-drug filling apparatus;
Fig. 22 is a diagram illustrating the relationship between the liquid pressure in the nozzle and supply of a drug containing solution;
Figs. 23A to 23C are schematic views of parts of processes for manufacturing the transdermal absorption sheet;
Figs. 24A to 24C are schematic views of parts of processes for manufacturing the transdermal absorption sheet;
Fig. 25 is a diagram illustrating a releasing process;
Fig. 26A is a plan view an original plate; and
Fig. 26B is a side view of the original plate.

### DETAILED DESCRIPTION OF THE PREFERRED EMBODIMENTS

Hereinafter, descriptions will be given of a preferred embodiment of the present invention in accordance with the accompanying drawings. The present invention will be described using the following preferred embodiment. However, many techniques may be used to modify the embodiment.

In the drawings, components represented by the same reference character are similar components having similar functions. Furthermore, in the present specification, when a numerical range is described using "to", numerical values for an upper limit and a lower limit described with "to" are also included in the numerical range.

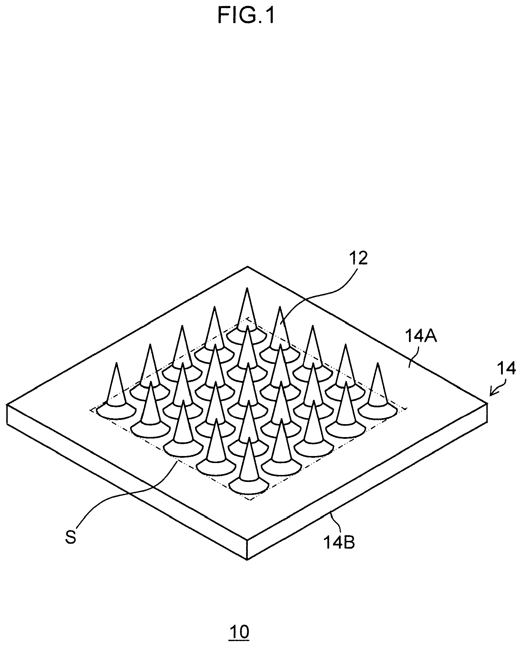

Fig. 1 is an entire perspective view of a transdermal absorption sheet. The transdermal absorption sheet 10 has a function of delivering a drug into a skin when attached to the skin. As illustrated in Fig. 1, the transdermal absorption sheet 10 is formed of a sheet portion 14 including a first main surface 14A (one surface) and a second main surface 14B (the other surface), and a plurality of needle-like protruding portions 12 arranged on the first main surface 14A of the sheet portion 14. The needle-like protruding portions 12 may be referred to as microneedles.

The needle-like protruding portions 12 each have a shape entirely tapered toward a side opposite to the sheet portion 14. For example, examples of the shape of the needle-like protruding portion 12 include a cone shape (including a pyramid and a circular cone), a combination shape of a truncated cone and a cone, and a combination shape of a truncated cone, a columnar shape, and a cone. The height of the needle-like protruding portion 12 is preferably within a range of 100 to 2000 µm, and more preferably, within a range of 200 to 1500 µm.

The sheet portion 14 has a flat shape as a whole so as to have a small thickness with respective to the two opposing large main surfaces (the first main surface 14A and the second main surface 14B). However, the main surfaces do not need to be completely flat. The sheet portion 14 in Fig. 1 has a rectangular shape in a plan view. However, the sheet portion 14 may have a polygonal shape, a circular shape, an oval shape, or the like. The thickness of the sheet portion 14 is preferably within a range of 10 to 2000 µm, and more preferably, within a range of 10 to 1000 µm.

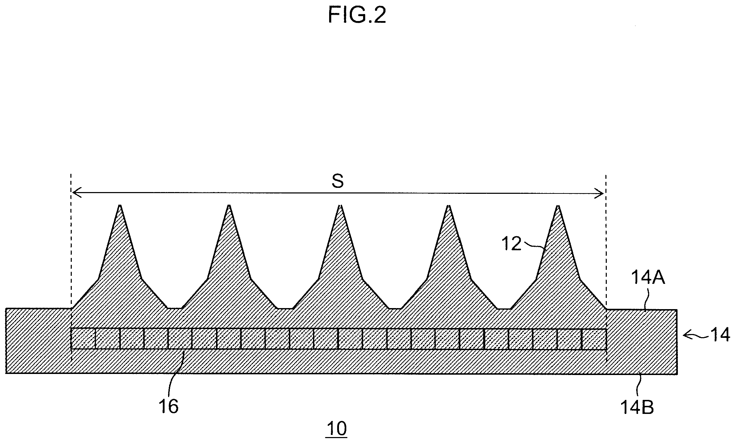

Fig. 2 is a cross-sectional view of the transdermal absorption sheet 10. As illustrated in Fig. 2, in the transdermal absorption sheet 10 of the present embodiment, the sheet portion 14 includes a sheet-like mesh structure body 16. Since the transdermal absorption sheet 10 includes the mesh structure body 16, the mesh structure body 16 functions as a reinforcement material of the sheet portion 14. The mesh structure body 16 can suppress a curl due to expansion/contraction of the sheet portion 14, and can prevent breakage of the sheet portion 14 due to impact at the time of puncture.

The sheet-like mesh structure body 16 refers to a shape which has two main surfaces each having an area larger than the thickness and which has a plurality of through holes connecting the two main surfaces to each other. The material of the mesh structure body 16 may be metal or resin. As the material, metal having good sterilizing properties and generating little eluted material is preferable, and stainless having high corrosion resistance, such as SUS (steel use stainless) 316L, SUS 316, SUS 304 (stipulated in JIS (Japanese Industrial Standard)) is particularly preferable.

As illustrated in Figs. 1 and 2, the mesh structure body 16 of the present embodiment has an area, in a plan view, including at least a part of a region S in which the plurality of needle-like protruding portions 12 are arranged. The region S in which the plurality of needle-like protruding portions 12 are arranged is a region surrounded by a virtual line indicated by a two-dot chain line which is in contact with the needle-like protruding portions 12 at the outermost peripheral part in Fig. 1. The expression that the mesh structure body 16 includes at least a part of the region S in which the plurality of needle-like protruding portions 12 are arranged refers to a state where at least a part of the region S overlaps with the mesh structure body 16 in a plan view. The part of the region S means 50% or more in area of the region S.

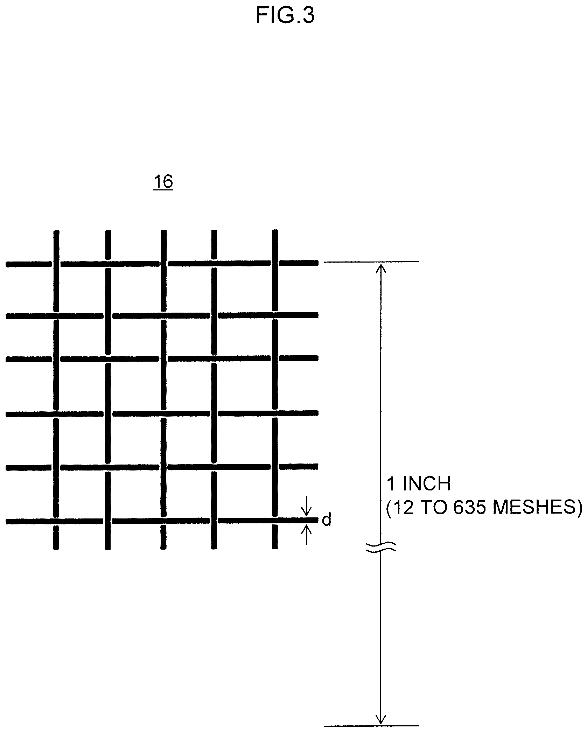

Depending on the material forming the mesh structure body 16, a woven net structure, a punched structure, a molded structure, or the like can be adopted as the mesh structure body 16. The woven net structure means a structure in which a plurality of linear members are woven to form through holes. The punched structure means a structure in which though holes are formed by punching a part of a flat member. The molded structure means a structure in which a material is poured into a mold, cured, and released from the mold to form through holes. As illustrated in Fig. 3, in the mesh structure body 16 having the woven net structure, the number of meshes per inch is preferably within a range of 12 to 635 meshes, and the wire diameter d is preferably within a range of 0.02 to 1.0 mm. The number of meshes per inch is preferably 12 to 635 meshes, and is more preferably 16 to 100 meshes. The wire diameter d is preferably within a range of 0.02 to 1.0 mm, and is more preferably within a range of 0.1 to 0.6 mm. The number of meshes per inch is represented by a unit "mesh".

The through holes of the mesh structure body 16 have no dead end. In contrast, a sintered body of metal particles, or a porous substrate formed of a fibrous filter has therein a dead end or a closed space (a space with no connection to the outside). If the mesh structure body 16 of the present embodiment is replaced with a thick porous material, a certain time is required to diffuse vapors in the porous material due to the presence of a dead end or a closed space, thereby causing a problem of increase in drying time. In contrast, in the mesh structure body 16 of the present embodiment, since vapors are diffused directly to the outside air through the through holes, drying is easily performed. In a porous substrate, the opening ratio is less than the porosity due to the presence of a dead end or a closed space. Here, the opening ratio means the ratio of the total opening area of the holes per unit area of the porous substrate surface. The porosity means the ratio of the total volume of the holes per unit volume of the entire porous substrate.

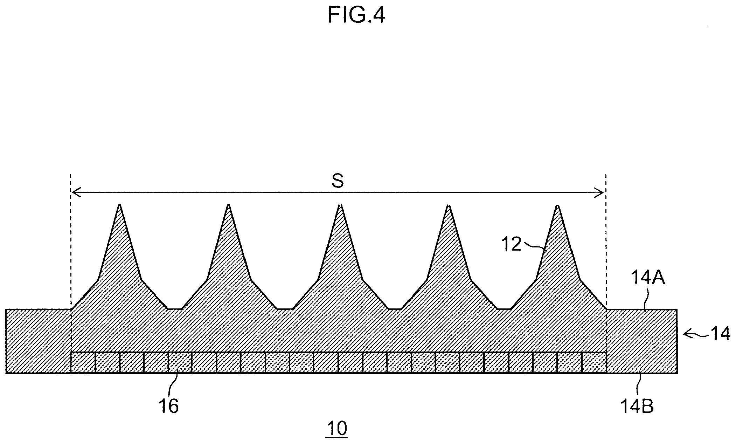

In the present embodiment, as illustrated in Fig. 2, the mesh structure body 16 is embedded in the sheet portion 14. Here, the term "embedded" means a state in which the mesh structure body 16 is not exposed from the sheet portion 14. However, the mesh structure body 16 is not limited to this structure. For example, as illustrated in Fig. 4, a part of the mesh structure body 16 may be exposed from the second main surface 14B of the sheet portion 14. The mesh structure body 16 included in the sheet portion 14 may have multiple layers but has preferably a single layer. When the mesh structure body 16 is embedded in the sheet portion, because an outside-air exposed area of a liquid forming the sheet portion can be maximized, a time for drying the liquid can be shortened. In contrast, when the mesh structure body 16 is partially embedded in the sheet portion and the amount of a liquid forming the sheet portion is reduced so as to make the other part of the mesh structure body 16 to be exposed from the sheet portion, because the amount of the liquid is small, the time for drying the liquid can be shortened.

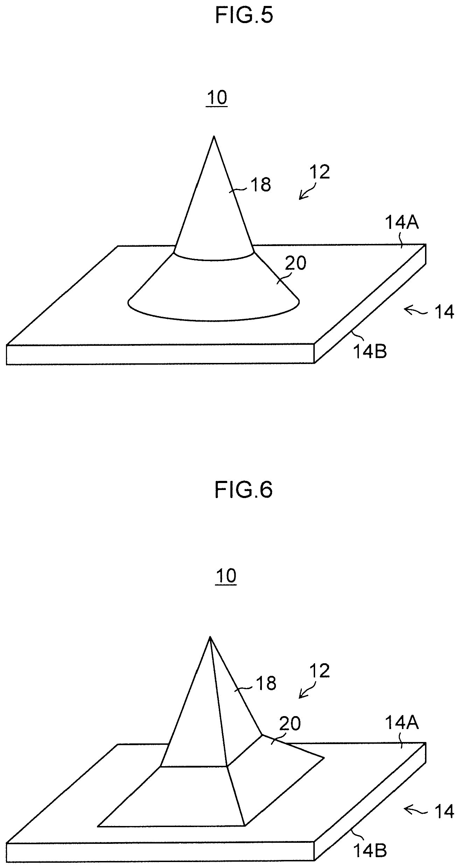

Next, the shape of the needle-like protruding portion 12 is described. Figs. 5 and 6 are partially enlarged views of the transdermal absorption sheet 10, and each illustrate the needle-like protruding portion 12.

As illustrated in Fig. 5, the transdermal absorption sheet 10 has a tapered needle portion 18, a truncated cone portion 20 connected with the needle portion 18, and the sheet-shape sheet portion 14 connected to the truncated cone portion 20. The needle-like protruding portion 12 is formed of the tapered needle portion 18 and the truncated cone portion 20.

A plurality of the truncated cone portions 20 (only one truncated cone portion 20 is illustrated in Fig. 5) are formed on the first main surface 14A of the sheet portion 14. The truncated cone portion 20 has two base surfaces and has a stereographic structure surrounded by conical surfaces. The base surface (the lower base) having a larger area of the two base surfaces of the truncated cone portion 20 is connected with the sheet portion 14. The base surface (the upper base) having a smaller area of the two base surfaces of the truncated cone portion 20 is connected with the needle portion 18. That is, of the two base surfaces of the truncated cone portion 20, the base surface farther from the sheet portion 14 has a smaller area.

The needle portion 18 has a tapered shape. That is, the needle portion 18 has a shape which is tapered from the larger base surface toward the tip. The base surface of the needle portion 18 is connected with the smaller base surface of the truncated cone portion 20. Thus, the needle-like protruding portion 12 formed of the needle portion 18 and the truncated cone portion 20 has a shape tapered from the sheet portion 14 toward the tip, as a whole. On the first main surface 14A of the sheet portion 14, 4 to 2500 needle-like protruding portions 12 are provided. However, the number of the needle-like protruding portions 12 is not limited to them.

In Fig. 5, the truncated cone portion 20 has a truncated cone shape, and the needle portion 18 has a cone shape. According to the degree of insertion of the needle portion 18 into a skin, the shape of the tip of the needle portion 18 may be changed, as appropriate, to a curved surface having a curvature radius of 0.01 to 50 µm, or a flat surface.

Fig. 6 illustrates the needle-like protruding portion 12 having another shape. In Fig. 6, the truncated cone portion 20 has a truncated quadrangular pyramid shape, and the needle portion 18 has a quadrangular pyramid shape.

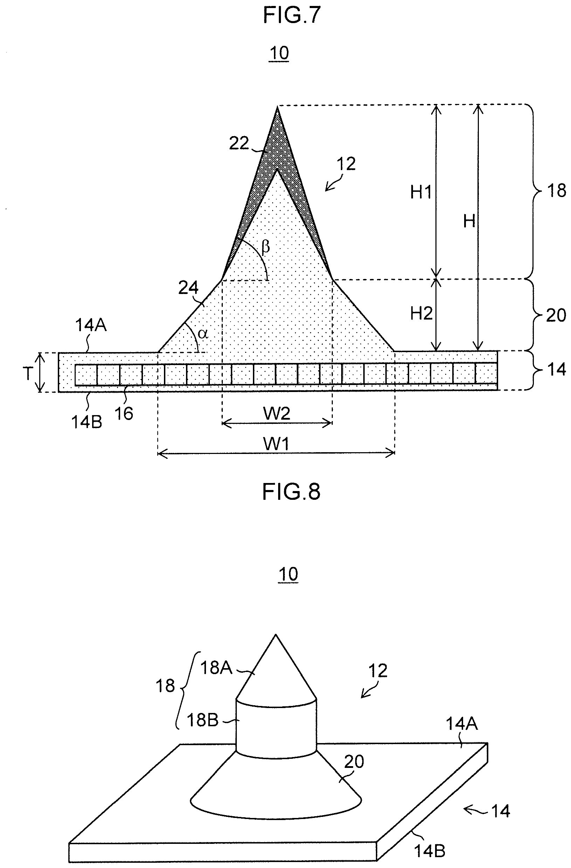

Fig. 7 is a cross-sectional view of the transdermal absorption sheet 10 illustrated in Figs. 5 and 6. As illustrated in Fig. 7, the transdermal absorption sheet 10 is formed of a first layer 22 containing a drug and a second layer 24 not containing a drug.

Here, the expression "containing a drug" means containing an amount of a drug large enough to exhibit effects of the drug at a time of puncture of a skin surface. The expression "not containing a drug" means not containing an amount of a drug large enough to exhibit effects of the drug, and includes "0", which indicates containing no drug, and amounts which do not exhibit effects of the drug. The first layer 22 containing a drug is formed at the tip (the tip of the needle portion 18) of the needle-like protruding portion 12. Since the first layer 22 is formed at the tip of the needle-like protruding portion 12, a drug can be efficiently delivered into a skin. Hereinafter, the expressions "containing a predetermined amount of a drug" and "not containing the predetermined amount of a drug" may be referred to, if necessary, as the expressions "containing a drug" and "not containing a drug", respectively.

The needle portion 18 is formed of the first layer 22 positioned at the tip side and containing a drug and the second layer 24 not containing a drug. The truncated cone portion 20 is formed of the second layer 24. The sheet portion 14 is formed of the second layer 24. That is, the sheet portion 14 and the truncated cone portion 20 are formed of the same material. The ratio of the first layer 22 and the second layer 24, which form the needle portion 18, the truncated cone portion 20, and the sheet portion 14, may be set as appropriate.

As described above, the thickness T of the sheet portion 14 is preferably within a range of 10 to 2000 µm, and more preferably, within a range of 10 to 1000 µm. The width W1 of the base surface (the lower base) on which the truncated cone portion 20 is in contact with the sheet portion 14 is preferably within a range of 100 to 1500 µm, and more preferably, within a range of 100 to 1000 µm. The width W2 of the base surface (the upper base) on which the truncated cone portion 20 is in contact with the needle portion 18 is preferably within a range of 100 to 1500 µm, and more preferably, within a range of 100 to 1000 µm. The width W1 and the width W2 are preferably within the respective above numerical ranges, and satisfy W1 > W2.

The sheet portion 14 includes the sheet-like mesh structure body 16. In the present embodiment, the mesh structure body 16 is embedded in the sheet portion 14.

As described above, the height H of the needle-like protruding portion 12 is preferably within a range of 100 to 2000 µm, and more preferably, within a range of 200 to 1500 µm. The ratio H1/H2 of the height H1 of the needle portion 18 to the height H2 of the truncated cone portion 20 is preferably within a range of 1 to 10, and more preferably, within a range of 1.5 to 8. In addition, the height H2 of the truncated cone portion 20 is preferably within a range of 10 to 1000 µm.

The angle α formed between a side surface of the truncated cone portion 20 and a plane parallel with the first main surface 14A of the sheet portion 14 is preferably within a range of 10 to 60°, and is more preferably, within a range of 20 to 50°. The angle β formed between a side surface of the needle portion 18 and a plane parallel with the upper base of the truncated cone portion 20 is preferably within a range of 45 to 85°, and is more preferably, within a range of 60 to 80°.

The angle β may be equal to the angle α, but it is preferable that the angle β is equal to or larger than the angle α. The reason for this is that it becomes easy to puncture a skin with the needle-like protruding portion 12.

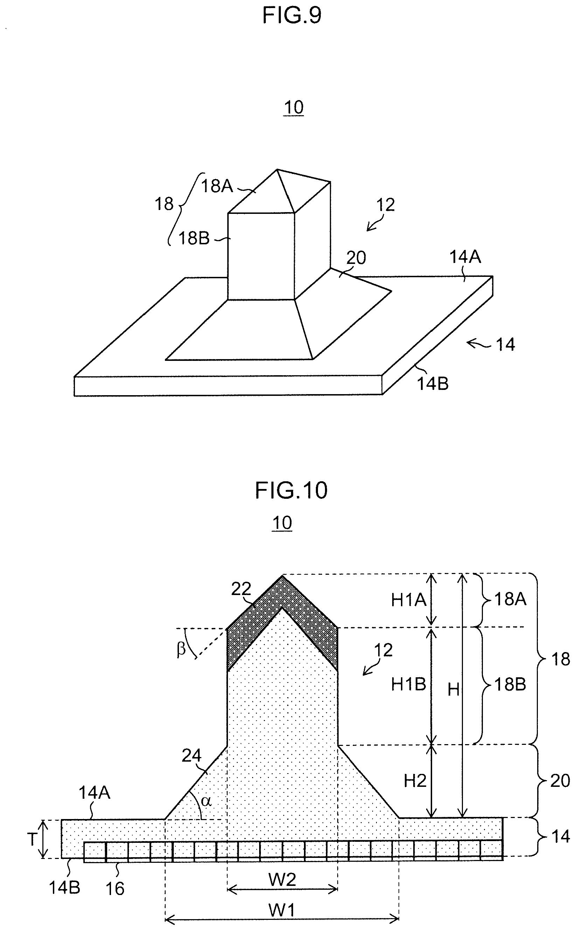

Figs. 8 and 9 each illustrate the needle-like protruding portion 12 having another shape. The transdermal absorption sheet 10 illustrated in Fig. 5 has the shape of the truncated cone portion 20 same as, and the shape of the needle portion 18 different from the transdermal absorption sheet 10 illustrated in Fig. 8. Likewise, the transdermal absorption sheet 10 illustrated in Fig. 6 has the shape of the truncated cone portion 20 same as, and the shape of the needle portion 18 different from the transdermal absorption sheet 10 illustrated in Fig. 9. The needle portion 18 illustrated in Figs. 8 and 9 has a tapered needle portion 18A and a cylindrical body portion 18B. The tapered needle portion 18A has a shape tapered from the larger base surface toward the tip. The cylindrical body portion 18B has two opposing base surfaces, the areas of which are substantially equal to each other. The larger base surface of the needle portion 18A is connected with one of the base surfaces of the body portion 18B. The other base surface of the body portion 18B is connected with the smaller base surface of the truncated cone portion 20.

In Fig. 8, the needle portion 18A has a cone shape and the body portion 18B has a cylindrical shape. In Fig. 9, the needle portion 18A has a quadrangular pyramid shape and the body portion 18B has a quadrangular pillar shape.

Since the needle portion 18 includes the body portion 18B, the needle portion 18 has a shape in which the cross-sectional area is constant in the direction away from the truncated cone portion 20. The needle portion 18A of the needle portion 18 has a shape tapered in the direction away from the body portion 18B. The needle portion 18 has a tapered shape as a whole. According to the insertion degree of the needle portion 18 into a skin, the shape of the tip of the needle portion 18 may be changed, as appropriate, to a curved surface having a curvature radius of 0.01 to 50 µm, or a flat surface.

Fig. 10 is a cross-sectional view of the transdermal absorption sheet 10 illustrated in Figs. 8 and 9. As illustrated in Fig. 10, the transdermal absorption sheet 10 is formed of the first layer 22 containing a drug and the second layer 24 not containing a drug. The first layer 22 containing a drug is formed at the tip (the tip of the needle portion 18) of the needle-like protruding portion 12. Since the first layer 22 is formed at the tip of the needle-like protruding portion 12, the drug can be efficiently delivered into a skin.

The needle portion 18 is formed of the first layer 22 positioned at its tip side and containing a drug, and the second layer 24 not containing a drug. The truncated cone portion 20 is formed of the second layer 24. The sheet portion 14 is formed of the second layer 24. The ratio of the first layer 22 and the second layer 24, which form the needle portion 18, the truncated cone portion 20, and the sheet portion 14, may be set as appropriate.

The thickness T of the sheet portion 14, the width W1 of the lower base of the truncated cone portion 20, the width W2 of the upper base of the truncated cone portion 20, the height H of the needle-like protruding portion 12, and the height H2 of the truncated cone portion 20 may be set as in the transdermal absorption sheet 10 illustrated in Fig. 7. The ratio H1/H2 of the height H1 of the needle portion 18 to the height H2 of the truncated cone portion 20 may be set as in the transdermal absorption sheet 10 illustrated in Fig. 7.

The sheet portion 14 includes the sheet-like mesh structure body 16. In the present embodiment, a part of the mesh structure body 16 is embedded in the sheet portion 14, and the other part of the mesh structure body 16 is exposed from the sheet portion 14.

Regarding the ratio H1B/H1A between the height H1B of the body portion 18B and the height H1A of the needle portion 18A, the ratio H1B/H1A is within a range of 0.1 to 4, and preferably, within a range of 0.3 to 2.

The angle α formed between a side surface of the truncated cone portion 20 and a plane parallel with the first main surface 14A of the sheet portion 14 is within a range of 10 to 60°, and preferably, within a range of 20 to 50°. The angle β formed between a side surface of the needle portion 18A and a plane parallel with the base surface of the body portion 18B is within a range of 45 to 85°, and preferably, within a range of 60 to 80°.

It is preferable that the angle β is equal to or larger than the angle α. The reason for this is that it becomes easy to puncture a skin with the needle-like protruding portion 12.

In the present embodiment, the transdermal absorption sheet 10 having the needle-like protruding portions 12 illustrated in Figs. 5, 6, 8, and 9 is exemplified. However, the needle-like protruding portions 12 of the transdermal absorption sheet 10 are not limited those shapes.

### (Mold)

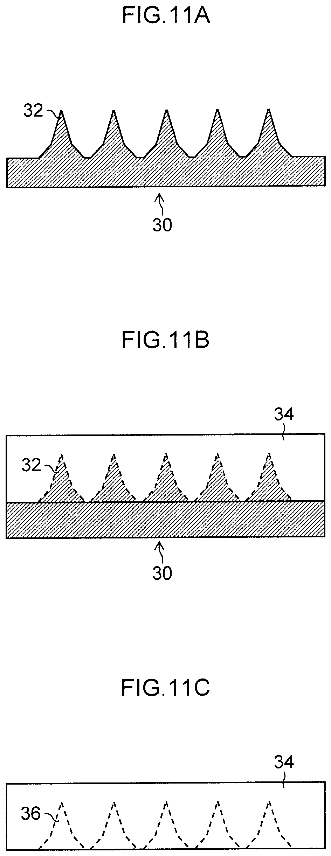

Figs. 11A to 11C are diagrams of processes for manufacturing a mold.

As illustrated in Fig. 11A, first, an original plate for manufacturing a mold for manufacturing the transdermal absorption sheet is manufactured.

There are two types of a method of manufacturing the original plate 30. In one method, a photoresist is applied onto an Si substrate, and then, exposure and development are carried out. Subsequently, etching such as RIE (reactive ion etching) is carried out to form a plurality of needle-like protruding portions 32 in an array on the surface of the original plate 30 such that the needle-like protruding portions 32 each have a shape same as the needle-like protruding portion of the transdermal absorption sheet. When etching such as RIE is carried out in order to form the needle-like protruding portions 32 on the surface of the original plate 30, the etching is carried out in an oblique direction while the Si substrate is being rotated. This enables forming of the needle-like protruding portions 32.

In the other method, processing is carried out on a metal substrate such as stainless, an aluminum alloy, and Ni by using a cutting tool such as a diamond cutting tool, to form a plurality of the needle-like protruding portions 32 in an array on the surface of the original plate 30.

Next, as illustrated in Fig. 11B, a mold 34 is manufactured using the original plate 30. To manufacture the mold 34, a method through Ni-electrocasting or the like is generally used. The original plate 30 includes the needle-like protruding portions 32 each having a cone shape or a pyramid shape (for example, a rectangular pyramid) with an acute tip. Thus, the shape of the mold 34 can be precisely transferred and the mold 34 can be removed from the original plate 30. Furthermore, there are four types of manufacturing methods which enable manufacture at low cost.

In the first method, a silicone resin obtained by adding a curing agent to PDMS (polydimethylsiloxane, for example, sylgard 184 manufactured by Dow Corning Corporation) is poured into the original plate 30, is subjected to heating processing at 100°C and cured, and then, the mold 34 is removed from the original plate 30. In the second method, an ultraviolet curing resin which is curable by irradiation with ultraviolet rays is poured into the original plate 30, and is irradiated with ultraviolet rays in a nitrogen atmosphere, and then, the mold 34 is removed from the original plate 30. In the third method, an organic solvent containing dissolved plastic resin such as polystyrene and PMMA (polymethyl methacrylate) is poured into the original plate 30 onto which a removing agent has been applied, is dried to vaporize the organic solvent, and is cured, and then, the mold 34 is removed from the original plate 30. In the fourth method, Ni-electrocasting is carried out to create a reversed product.

Accordingly, the mold 34 in which the needle-like recessed portions 36 each having the reversed shape of the needle-like protruding portion 32 of the original plate 30 are two-dimensionally arranged is manufactured. Fig. 11C illustrates the mold 34 manufactured in this way. The shape of each needle-like protruding portion 32 of the original plate 30 is same as the shape of each needle-like protruding portion of the transdermal absorption sheet. Thus, as illustrated in Fig. 11C, the mold 34 including the plurality of needle-like recessed portions 36, which is a reversed mold of the needle-like protruding portions of the transdermal absorption sheet is manufactured. Any one of the above four methods can manufacture the mold 34 easily many times.

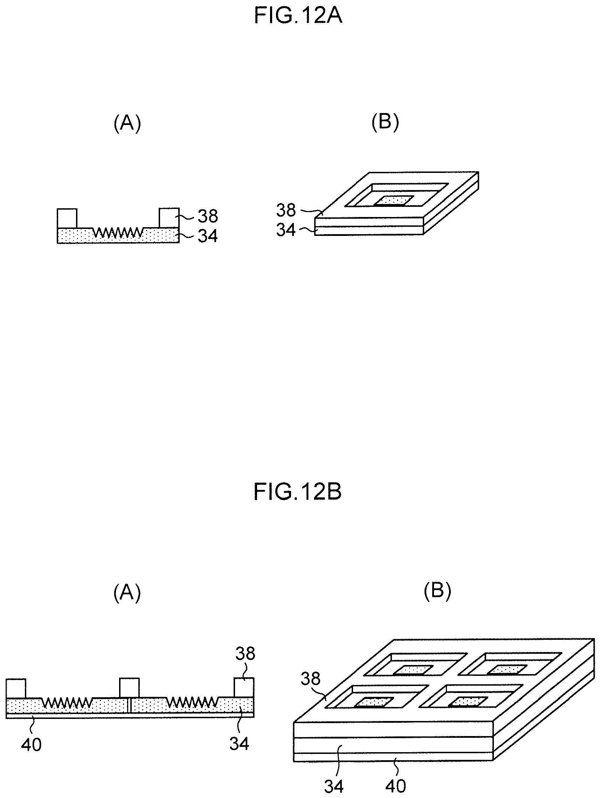

Figs. 12A and 12B are diagrams illustrating the states where a frame 38 is set in the mold 34 manufactured in Fig. 11C. Part (A) and Part (B) in Fig. 12A are diagrams illustrating the state where the frame 38 is provided on the periphery of the surface of one mold 34. Part (A) and Part (B) in Fig. 12B are diagrams illustrating the state where the frame 38 is provided on the periphery of the connected molds 34 and inside of the connected molds 34. Since the frame 38 is provided, a solution of a polymer resin (hereinafter, also referred to as "polymer solution") is prevented from flowing out to the outside of the mold(s) 34 when forming a functional film so as to have a desired film thickness.

At that time, it is preferable that the level difference between the mold(s) 34 and the frame 38 is 50 µm to 10 mm. In the configurations in Figs. 12A and 12B, the mold(s) 34 and the frame 38 are separatable from each other. However, the mold(s) 34 and the frame 38 may be integrated with each other. In the case where the mold(s) 34 and the frame 38 are separatable, the frame 38 can be removed in a drying step and a releasing step posterior to a filling step.

As illustrated in Fig. 12B, a plurality of the molds 34 are connected to each other with an adhesive on the substrate 40. The frame 38 is set on the periphery of the connected molds 34 and inside of the connected molds 34.

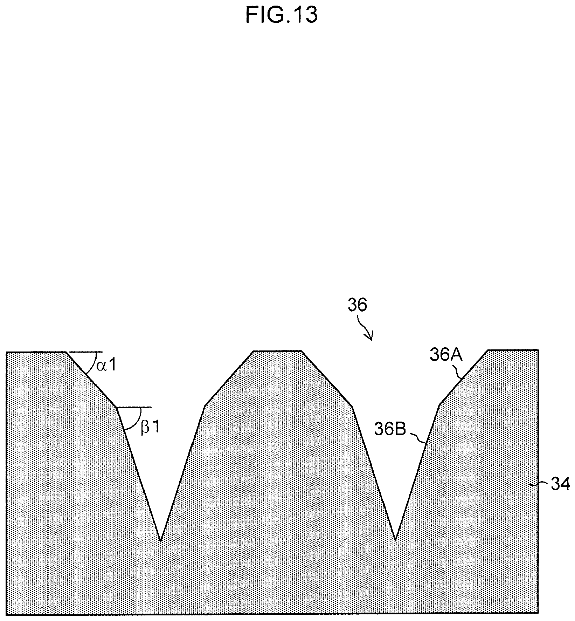

Fig. 13 is a partially enlarged view of the mold 34. The needle-like recessed portion 36 is provided with a tapered inlet portion 36A which is narrowed from the surface of the mold 34 toward the depth direction and a tip recessed portion 36B which is tapered in the depth direction. The angle α1 of the taper of the inlet portion 36A basically corresponds to the angle α formed between the side surface of the truncated cone portion and the sheet portion of the transdermal absorption sheet. The angle β1 of the taper of the tip recessed portion 36B basically corresponds to the angle β formed between the side surface of the needle portion and the upper base of the truncated cone portion.

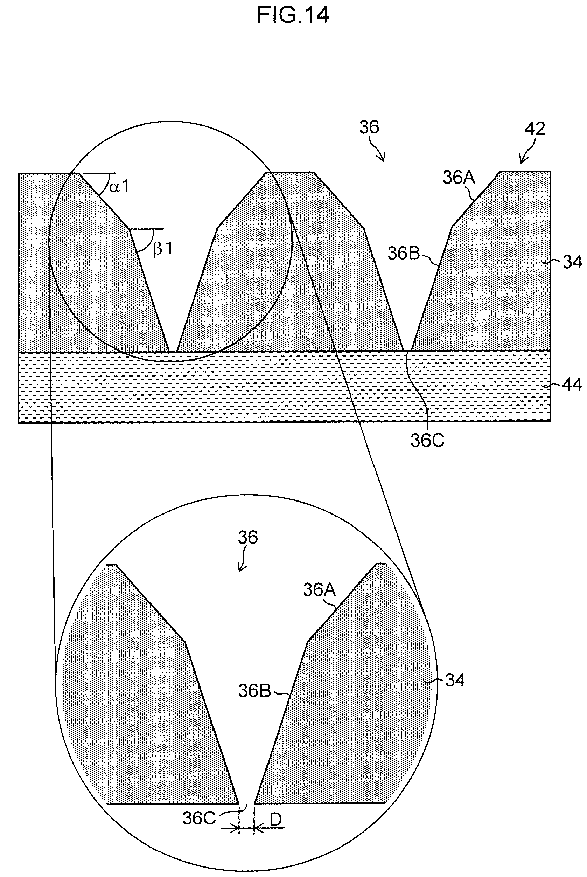

Fig. 14 illustrates a form of a mold composite body 42 which is more preferred in carrying out the manufacturing method of the transdermal absorption sheet. As illustrated in Fig. 14, the mold composite body 42 is formed of the mold 34 in which through holes 36C are formed at the tips of the needle-like recessed portions 36, and a gas permeable sheet 44 which is attached to the through hole 36C side of the mold 34 and which is made of a material permeable to gas but not permeable to liquid. The through holes 36C connect the tips of the needle-like recessed portions 36 to the outside air through the gas permeable sheet 44 interposed therebetween. The tip of the needle-like recessed portion 36 means a side which is tapered in the depth direction of the mold 34, which is a side opposite to the side in which a liquid drug and a base material liquid are filled.

By using the above mold composite body 42, only air which exists in the needle-like recessed portions 36 can be released through the through holes 36C while the solution of the transdermal absorption material filled in the needle-like recessed portions 36 does not permeate. The transfer performance of the shapes of the needle-like recessed portions 36 onto the transdermal absorption material can be improved, and the more acute needle-like protruding portions can be formed.

The diameter D of the through hole 36C is preferably within a range of 1 to 50 µm. With this range, air can be easily removed and the tip portions of the needle-like protruding portions of the transdermal absorption sheet can be formed to be acute. As the gas permeable sheet 44 formed of a material which is permeable to gas but not permeable to liquid, for example, Poreflon (registered trademark of Sumitomo Electric Industries, Ltd.) can be suitably used.

As the material used for the mold 34, an elastic raw material and a metallic raw material can be used. In particular, an elastic material is preferable. A raw material having a high gas-permeability is more preferable. The oxygen permeability as representative of the gas permeability is preferably higher than 1×10⁻¹² (mL/s·m·Pa), and more preferably, higher than 1×10⁻¹⁰ (mL/s·m·Pa). With the gas permeability within the above range, air present in the needle-like recessed portions 36 of the mold 34 can be removed from the mold 34 side. The transdermal absorption sheet having few defects can be manufactured. Specific examples of such material include materials obtained by melting silicone resin (for example, Sylgard 184, 1310ST), ultraviolet curing resin, or plastic resin (for example, polystyrene or PMMA (polymethylmethacrylate)), and materials obtained by dissolving any of the above resins into a solvent. Among these materials, silicone rubber-based materials can be suitably used because of the durability thereof against transfers using repeated pressurization and the good releaseability thereof from the raw material. Furthermore, examples of the metallic raw material include Ni, Cu, Cr, Mo, W, Ir, Tr, Fe, Co, MgO, Ti, Zr, Hf, V, Nb, Ta, α-aluminum oxide, zirconium oxide, stainless steel (for example, a STAVAX material, STAVAX: registered trademark of Bohler Uddeholm AG), and alloys thereof. For the material of the frame 38, a material similar to the material of the mold 34 may be used.

### (Polymer solution)

A polymer solution which is a solution of polymer resin used in the present embodiment is described.

In the present embodiment, the term "polymer solution containing a drug" or "solution containing a drug" means a polymer solution containing a predetermined amount of a drug, or a solution containing a predetermined amount of a drug. The term "polymer solution not containing a drug" or "solution not containing a drug" means a polymer solution not containing the predetermined amount of a drug or a solution not containing the predetermined amount of a drug.

In addition, in some cases, a polymer solution containing a drug is referred to as a liquid drug, and a polymer solution not containing a drug is referred to as a base material liquid.

Whether a solution contains the predetermined amount of a drug is determined based on whether the solution exerts effects of the drug at a time of puncture of a body surface. Thus, the expression "containing the predetermined amount of a drug" means containing an amount of a drug large enough to exhibit effects of the drug at a time of puncture of a body surface. The expression "not containing the predetermined amount of a drug" means not containing an amount of a drug large enough to exert effects of the drug, the drug amount of which is within in a range from "0", which indicates containing no drug, to an amount which exerts no drug effect.

It is preferable that a biocompatible resin is used as a raw material for the resin polymer used for the polymer solution. It is preferable to use, as such a resin, sugar such as glucose, maltose, pullulan, sodium chondroitin sulfate, sodium hyaluronate, hydroxyethyl starch, or hydroxypropyl cellulose, protein such as gelatin, polylactate, or a biodegradable polymer such as a lactic acid-glycollic acid copolymer. Among these resins, a gelatin-based material has an adhesion with many base materials and has a high gel strength as materials to be gelated. Thus, the gelatin-based material can be suitably used during a releasing step, which will be described later, because the material can be brought into tight contact with the base material to allow the polymer sheet to be released off from the mold using the base material. It is preferable that the concentration of the resin is set such that 10 to 50% by mass of resin polymer is contained in the polymer solution not containing a drug, though the concentration depends on the type of the material. Additionally, a solvent used for dissolution may be other than hot water as long as the solvent has volatility, and methylethylketone, alcohol, or the like may be used. A drug to be supplied to the inside of a human body may concurrently be dissolved into a solution of the polymer resin in accordance with the application. It is preferable that the polymer concentration (the concentration of a polymer excluding a drug in a case where the drug itself is a polymer) of the polymer solution containing a drug is 0 to 40% by mass.

In a method of preparing the polymer solution, when a water-soluble polymer (gelatin or the like) is used, water-soluble powder may be dissolved into water, and after the dissolution, a drug may be added to the solution. Alternatively, water-soluble polymer powder may be poured and dissolved in a liquid containing a drug dissolved therein. If the polymer is difficult to dissolve into water, the polymer may be dissolved on heating. The temperature may be selected, as appropriate, depending on the type of the polymer material, but the solution is preferably heated at approximately 60°C or less. The viscosity of the solution of the polymer resin is preferably 100 Pa·s or less, and more preferably 10 Pa·s or less for the solution containing a drug. The viscosity of the solution of the polymer resin is preferably 2,000 Pa·s or less, and more preferably 1,000 Pa·s or less for the solution not containing a drug. Appropriate adjustment of the viscosity of the solution of the polymer resin facilitates injection of the solution into the needle-like recessed portions of the mold. The viscosity of the solution of the polymer resin can be measured with a capillary viscometer, a falling ball type viscometer, a rotary viscometer, or a vibration type viscometer, for example.

### (Drug)

The drug contained in the polymer solution is not limited to a particular drug as long as the drug has a material having bioactivity. The drug is preferably selected from the group consisting of peptide, protein, nucleic acid, polysaccharide, a vaccine, a medical compound, and a cosmetic component. The medical compound preferably belongs to a water-soluble low-molecular-weight compound. Here, the low-molecular-weight compound means a compound having a molecular amount of several hundreds to several thousands. Here, the medical compound means a compound used for diagnosis, treatment, or preventing a disease of a human.

### (Method of manufacturing transdermal absorption sheet)

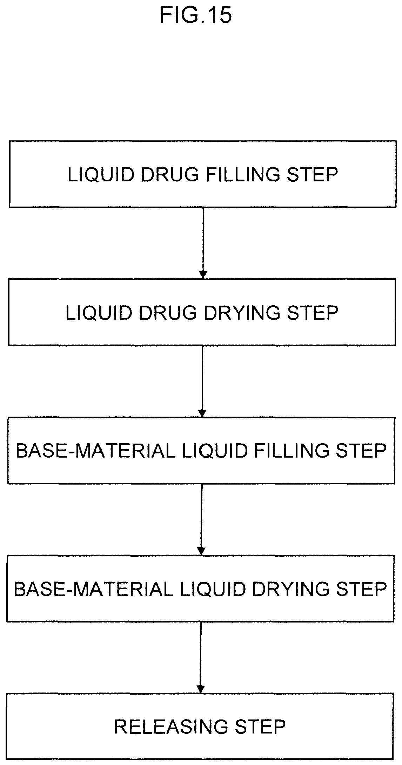

The method of manufacturing the transdermal absorption sheet of the present embodiment includes at least five steps of a liquid drug filling step, a liquid drug drying step, a base-material liquid filling step, a base-material liquid drying step, and a releasing step, in this order, as illustrated in Fig. 15.

### (Liquid drug filling step)

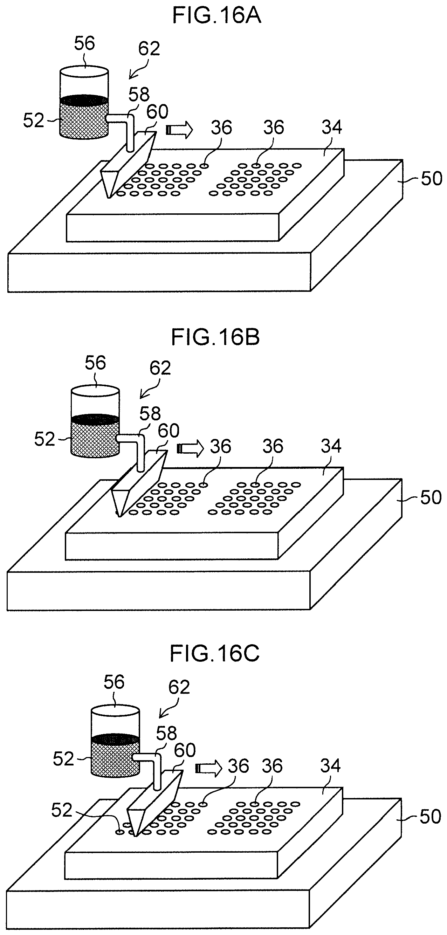

The method of manufacturing the transdermal absorption sheet using the mold 34 is described. As illustrated in Fig. 16A, the mold 34 having the two-dimensionally arranged needle-like recessed portions 36 is placed on a base 50. Two sets of the plurality of needle-like recessed portions 36, each set including 5×5 two-dimensionally arranged needle-like recessed portions 36, are formed in the mold 34. A liquid supply apparatus 62 is prepared which has a liquid feeding tank 56 housing a liquid drug 52, which is a polymer solution containing a drug, a pipe 58 connected to the liquid feeding tank 56, and a nozzle 60 connected to a tip of the pipe 58. The liquid drug 52 is discharged from the tip of the nozzle 60.

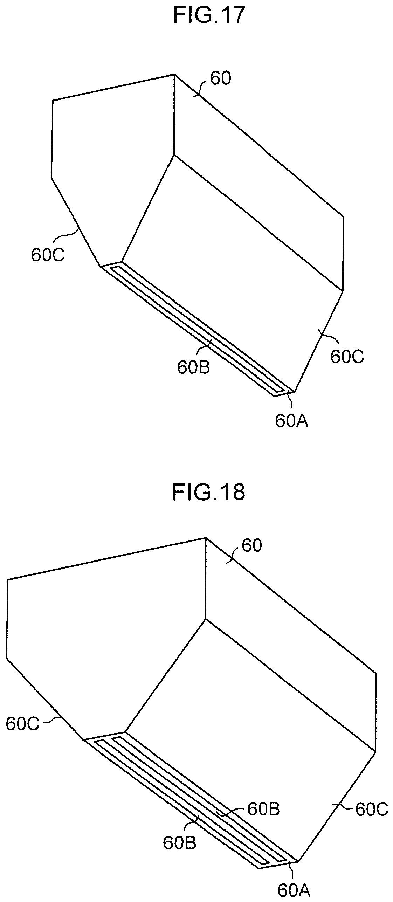

Fig. 17 illustrates a schematic perspective view of the tip of the nozzle. As illustrated in Fig. 17, the nozzle 60 includes a lip portion 60A which is a flat surface at the tip thereof, a slit-shaped opening 60B, and two inclined surfaces 60C which are spread in a direction away from the opening 60B along the lip portion 60A. For example, a plurality of needle-like recessed portions 36, which constitute one line, can be simultaneously filled with the liquid drug 52 by the slit-shaped opening 60B. The size (the length and the width) of the opening 60B is selected, as appropriate, in accordance with the number of the needle-like recessed portions 36 to be filled at a time.

When the opening 60B has a long length, the increased number of needle-like recessed portions 36 can be filled with the liquid drug 52 at a time. Thus, productivity can be improved.

Fig. 18 illustrates a schematic perspective view of a tip of another nozzle. As illustrated in Fig. 18, the nozzle 60 includes the lip portion 60A which is a flat surface at the tip thereof, two slit-shaped openings 60B, and two inclined surfaces 60C which are spread in a direction away from the openings 60B along the lip portion 60A. For example, a plurality of needle-like recessed portions 36, which constitute two lines, can be simultaneously filled with the liquid drug 52 containing a drug by the two openings 60B.

As a material used for the nozzle 60, an elastic raw material or a metallic raw material may be used. For example, Teflon (registered trademark), stainless steel, titanium, or the like may be used.

With reference to Fig. 16B, the filling step is described. As illustrated in Fig. 16B, the position of the opening 60B in the nozzle 60 is adjusted above the needle-like recessed portions 36. Since the nozzle 60 to discharge the liquid drug 52 is pressed against the mold 34, the lip portion 60A of the nozzle 60 is in contact with the surface of the mold 34. The liquid drug 52 is supplied from the liquid supply apparatus 62 to the nozzle 60, the liquid drug 52 is supplied from the opening 60B in the nozzle 60 to the mold 34, and the liquid drug 52 fills the needle-like recessed portions 36. In the present embodiment, the plurality of needle-like recessed portions 36, which constitute one line, are simultaneously filled with the liquid drug 52. However, the present disclosure is not limited to this configuration. The needle-like recessed portions 36 may be filled with the liquid drug 52 one by one. Alternatively, the plurality of needle-like recessed portions 36, which constitute multiple lines, can be simultaneously filled with the liquid drug 52 at a time by using the nozzle 60 illustrated in Fig. 18.

When the mold 34 is formed of a raw material having gas permeability, the liquid drug 52 can be sucked from the rear surface of the mold 34, thereby promoting filling of the needle-like recessed portions 36 with the liquid drug 52.

After the filling step in Fig. 16B, while causing the lip portion 60A of the nozzle 60 to be in contact with the front surface of the mold 34, the liquid supply apparatus 62 is caused to scan relatively in the longitudinal direction and the vertical direction of the opening 60B, as illustrated in Fig. 16C. The nozzle 60 is caused to scan over the mold 34, and the nozzle 60 is caused to move to the needle-like recessed portions 36 which have not filled with the liquid drug 52. The position of the opening 60B in the nozzle 60 is adjusted to be above the needle-like recessed portions 36. The present embodiment has been described with taking a case in which the nozzle 60 is caused to scan, as an example. However, the mold 34 may be caused to scan.

Since the nozzle 60 is caused to scan over the mold 34 while causing the lip portion 60A of the nozzle 60 to be in contact with the front surface of the mold 34, the nozzle 60 can scrape off the liquid drug 52 remaining on the front surface of the mold 34 except in the needle-like recessed portions 36. This enables the liquid drug 52 containing a drug to be prevented from remaining on the mold 34 except in the needle-like recessed portions 36. In addition, in the present embodiment, the nozzle 60 is arranged such that the inclined surfaces 60C are orthogonal to the scanning direction, which is indicated by the arrow. Accordingly, the nozzle 60 can smoothly scan over the mold 34.

In order to reduce damage to the mold 34 and to suppress deformation of the mold 34 due to compression as much as possible, it is preferable that the pressing pressure with which the nozzle 60 is pressed against the mold 34 at a time of scanning is controlled. For example, it is preferable that the pressing force of the nozzle 60 against the mold 34 and the press-in distance of the nozzle 60 into the mold 34 are controlled. Furthermore, in order to prevent the liquid drug 52 from remaining on the mold 34 except in the needle-like recessed portions 36, it is desired that at least one of the mold 34 and the nozzle 60 is formed of a flexible, elastically deformable material.

The 5×5 two-dimensionally arranged needle-like recessed portions 36 are filled with the liquid drug 52 by repeating the filling step in Fig. 16B and the moving step in Fig. 16C. When the 5×5 two-dimensionally arranged needle-like recessed portions 36 are filled with the liquid drug 52, the liquid supply apparatus 62 is moved to the adjacent 5×5 two-dimensionally arranged needle-like recessed portions 36, and the filling step in Fig. 16B and the moving step in Fig. 16C are repeated. The adjacent 5×5 two-dimensionally arranged needle-like recessed portions 36 are also filled with the liquid drug 52.

The above-described filling step and moving step may be performed by a mode (1) in which the needle-like recessed portions 36 are filled with the liquid drug 52 while the nozzle 60 is being caused to scan or a mode (2) in which, during scanning by the nozzle 60, the nozzle 60 is temporarily stopped above the needle-like recessed portions 36 to fill the needle-like recessed portions 36 with the liquid drug 52, and the nozzle 60 starts to scan again after the filling. In the filling step and the moving step, the lip portion 60A of the nozzle 60 is pressed against the front surface of the mold 34. It is preferable that the amount of the liquid drug 52 to be discharged from the liquid supply apparatus 62 is equal to the total volume of the plurality of needle-like recessed portions 36 to be filled in the mold 34. The liquid drug 52 can be prevented from remaining on the front surface of the mold 34 except in the needle-like recessed portions 36, and loss of the drug can be reduced.

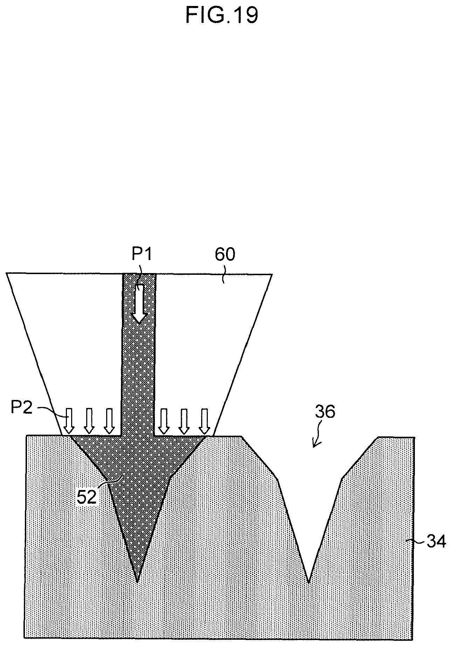

Fig. 19 is a partially enlarged view of the tip of the nozzle 60 and the mold 34 when the needle-like recessed portions 36 are being filled with the liquid drug 52. As illustrated in Fig. 19, filling of the needle-like recessed portions 36 with the liquid drug 52 can be promoted by applying a pressuring force P1 to the inside of the nozzle 60. Moreover, when the needle-like recessed portions 36 are filled with the liquid drug 52, a pressing force P2 with which the nozzle 60 is brought into contact with the front surface of the mold 34 is preferably set equal to or higher than the pressuring force P1 in the nozzle 60. Satisfying "pressing force P2 ≥ pressuring force PI" can suppress leakage of the liquid drug 52 from the needle-like recessed portions 36 to the front surface of the mold 34.

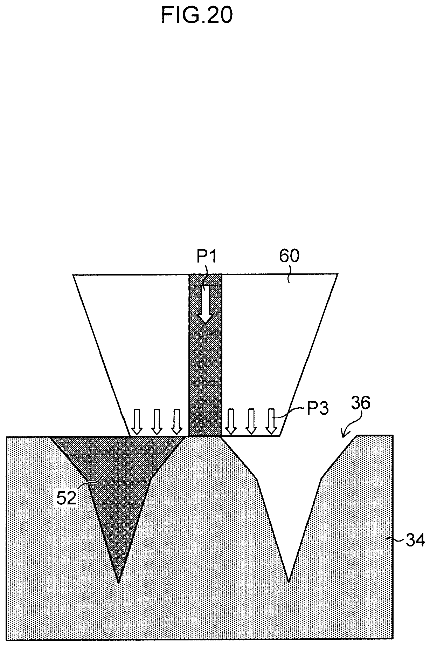

Fig. 20 is a partially enlarged view of the tip of the nozzle 60 and the mold 34 during movement of the nozzle 60. When the nozzle 60 is caused to scan relatively to the mold 34, a pressing force P3 with which the nozzle 60 is brought into contact with the front surface of the mold 34 is preferably set smaller than the pressing force P2 with which the nozzle 60 is brought into contact with the front surface of the mold 34 while filling is performed. This is intended to reduce damage to the mold 34 and to suppress deformation of the mold 34 due to compression.

It is preferable that the lip portion 60A of the nozzle 60 is parallel with the front surface of the mold 34. The posture of the nozzle 60 may be controlled by providing a joint driving mechanism to an attachment portion of the nozzle 60.

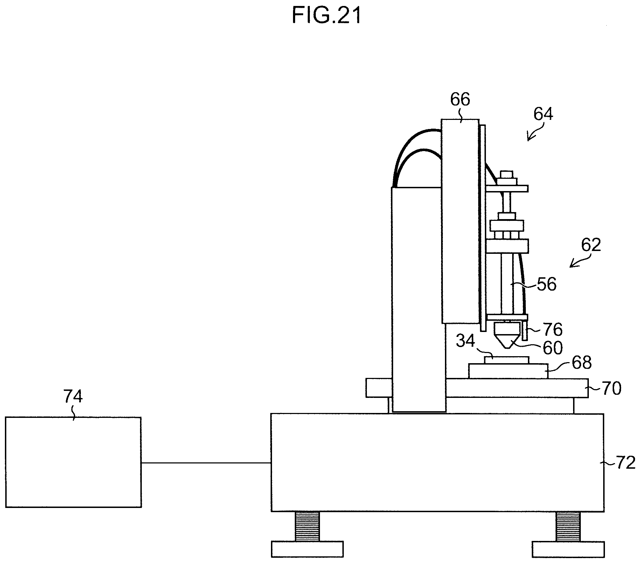

It is preferable that the pressing force of the nozzle 60 against the mold 34 and/or the push-in distance is controlled by driving the nozzle 60 in the Z-axis direction depending on the surface shape of the mold 34. Fig. 21 is a schematic configuration diagram of the liquid-drug filling apparatus 64 that can control the pressing force and/or the push-in distance. The liquid-drug filling apparatus 64 includes: a liquid supply apparatus 62 having the liquid feeding tank 56 housing the liquid drug and the nozzle 60 attached to the liquid feeding tank 56; a Z-axis driving unit 66 that drives the liquid feeding tank 56 and the nozzle 60 in the Z-axis direction; a suction base 68 on which the mold 34 is placed; an X-axis driving unit 70 that drives the suction base 68 in the X-axis direction; a pedestal 72 that supports the apparatus; and a control system 74.

A case where the pressing force is controlled to be constant is described. The Z-axis driving unit 66 brings the nozzle 60 into close with the mold 34 such that the nozzle 60 reaches a Z-coordinate, at which a desired pressing force is obtained. While the X-axis driving unit 70 causes the nozzle 60 in contact with the mold 34 to scan, the pressing force is made constant by controlling the Z-axis coordinate, and the liquid drug 52 is discharged from the nozzle 60. A method of measuring a contact pressure is not limited to a particular method. For example, various load cells may be used under the suction base 68, or in place of the suction base 68. The load cell means a measurement apparatus capable of measuring a compression force in a thickness direction. It is possible that the pressing force can be controlled to be an arbitrary constant pressure in a range of 1 to 1000 kPa against the mold 34.

A case where the press-in distance is controlled to be constant is described. The surface shape of the mold 34 is measured in advance before the mold 34 is brought into contact with the nozzle 60. While the X-axis driving unit 70 causes the nozzle 60 in contact with the mold 34 to scan, a value obtained by offsetting the Z-axis coordinate is fed back to the Z-axis driving unit 66 so as to obtain a desired press-in distance with respect to the surface shape of the mold 34, and the liquid drug 52 is discharged.

A method for shape measurement is not limited to a particular method. For example, an optical measuring apparatus such as a non-contact type laser displacement gauge 76, a contact type stylus step profiler, or the like may be used. In addition, the posture of the nozzle 60 in the slit direction may be controlled according to the surface shape of the mold 34. It is preferable that the press-in distance is controlled within a range of 1 to 15% with respect to the thickness of the mold 34. As a result of performing operation while the Z-axis driving unit 66 controls the distance between the nozzle 60 and the mold 34 in the Z-axis direction according to the shape of the mold 34, a compression deformation rate is uniformized and the accuracy of a filling amount can be improved.

Regarding control of the pressing force and the press-in distance, it is preferable that the pressing force is controlled when the press-in distance is small, and it is preferable that the press-in distance is directly controlled when the press-in distance is large.

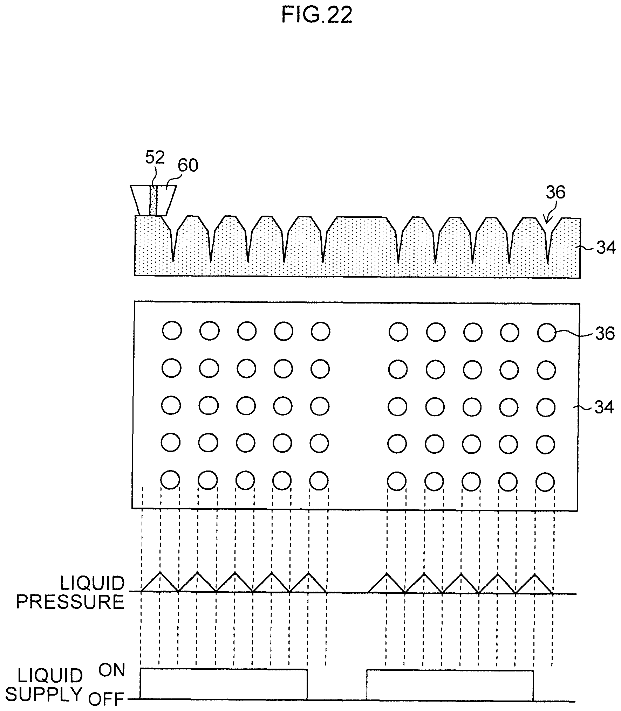

Fig. 22 is a diagram illustrating the relation between the liquid pressure in the nozzle and supply of the solution containing a drug. As illustrated in Fig. 22, supply of the liquid drug 52 starts before the nozzle 60 is positioned above the needle-like recessed portions 36. This is intended to reliably fill the needle-like recessed portions 36 with the liquid drug 52. The liquid drug 52 is continuously supplied to the mold 34 until the filling of the plurality of 5×5 needle-like recessed portions 36 is completed. Supply of the liquid drug 52 to the mold 34 is stopped before the nozzle 60 is moved above the fifth line of the needle-like recessed portions 36. This can prevent the liquid drug 52 from overflowing from the needle-like recessed portions 36. When the supply of the liquid drug 52 starts, the liquid pressure in the nozzle 60 becomes high in an area where the nozzle 60 is not positioned above the needle-like recessed portions 36. On the other hand, when the nozzle 60 is positioned above the needle-like recessed portions 36, the needle-like recessed portions 36 are filled with the liquid drug 52 and the liquid pressure in the nozzle 60 becomes low. Thus, the liquid pressure repeatedly varies.

When filling of the plurality of 5×5 needle-like recessed portions 36 is completed, the nozzle 60 is moved to the adjacent plurality of 5×5 needle-like recessed portions 36. Regarding the liquid supply, it is preferable that supply of the liquid drug 52 is stopped while the nozzle 60 is moved to the adjacent plurality of 5x5 needle-like recessed portions 36. There is a distance between the fifth line of the needle-like recessed portions 36 and the next first line of the needle-like recessed portions 36. When supply of the liquid drug 52 is continued while the nozzle 60 is scanning between the fifth line and the next first line, the liquid pressure in the nozzle 60 may be excessively high. As a result, the liquid drug 52 may flow out from the nozzle 60 onto an area other than the needle-like recessed portions 36 of the mold 34. In order to prevent this, it is preferable that supply of the liquid drug 52 is stopped.

To perform the liquid drug filling, it is preferable that the tip of the nozzle 60 having been cleaned is used. When there is a matter adhered onto the surface of the lip portion 60A of the nozzle 60 before performing filling, the accuracy of the filling amount with the liquid drug 52 may be deteriorated. Cleaning is generally performed by wiping with a non-woven cloth. Cleaning can be efficiently performed by moistening a non-woven cloth with water or a solvent in wiping. When the nozzle 60 is separated from the mold 34 after performing the filling with the liquid drug 52, the liquid drug may remain on the front surface of the mold 34. After completing the filling of the needle-like recessed portions 36, suck back control for sucking the liquid drug through the opening 60B of the nozzle 60 is performed to suck the excessively discharged liquid drug 52, thereby reducing the liquid residue on the front surface of the mold 34.

In the liquid drug filling step, the needle-like recessed portions 36 can be filled with the liquid drug 52 by using the mold composite body 42 illustrated in Fig. 14 and performing sucking from the through holes 36C side. It is because unfavorable variation in liquid drug content occurs, particularly, when bubbles are introduced into the liquid drug 52.

After completion of the filling of the needle-like recessed portions 36 with the liquid drug 52, the process proceeds to the liquid drug drying step, the base-material liquid filling step, the base-material liquid drying step, and the releasing step.

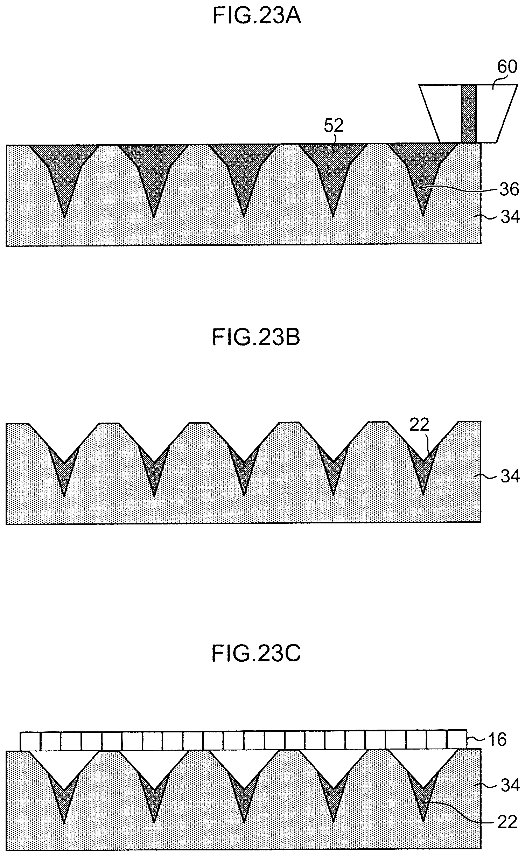

As illustrated in Fig. 23A, in the liquid drug filling step, the needle-like recessed portions 36 of the mold 34 are filled with the liquid drug 52 by the nozzle 60. The liquid drug filling step is performed by the above method.

### (Liquid drug drying step)

As illustrated in Fig. 23B, in the liquid drug drying step, the liquid drug 52 is dried and solidified to form the first layers 22 containing drugs in the needle-like recessed portions 36.

The liquid drug drying step is a step for drying the liquid drug 52 filling the needle-like recessed portions 36 of the mold 34, and forming the first layers 22 containing drugs so as to be localized at the tips of the needle-like recessed portions 36. In the present embodiment, it is preferable that the liquid drug drying step is performed in an environment having a temperature of 1 to 10°C.

By controlling the temperature and humidity conditions in the liquid drug drying step to optimize the drying speed, the liquid drug 52 is prevented from being fixed on the wall surfaces, in the needle-like recessed portions 36, of the mold 34. Drying proceeds while the liquid drug 52 is being concentrated in the tips of the needle-like recessed portions 36 due to drying. For example, in an environment having a temperature of 23°C and a relative humidity of 40 to 60%RH (RH: relative humidity), the drying speed is high. Thus, it may be difficult to localize the liquid drug 52 at the tips of the needle-like recessed portions 36 because the liquid drug 52 is fixed onto the wall surfaces, in the needle-like recessed portions 36, of the mold 34.

As a result of performing the liquid drug drying step in an environment having a temperature of 1 to 10°C, the drying speed of the liquid drug 52 can be lowered. Thus, the liquid drug 52 can be localized at the tips of the needle-like recessed portions 36 without fixing the liquid drug 52 onto the wall surfaces of the mold 34. In the liquid drug drying step in an environment having a temperature of 1 to 10°C, when the humidity is high, the drying speed of the liquid drug 52 is low, thereby causing reduction in productivity. Accordingly, when the liquid drug drying step is performed in an environment having a temperature of 1 to 10°C, an environment having a relative humidity of 1 to 59% is preferably provided, and more preferably, an environment having a relative humidity of 21 to 39% is provided. In an environment having a temperature and humidity range in which the temperature 1 to 10°C and the relative humidity is 1 to 59%, the liquid drug 52 can be localized at the tips of the needle-like recessed portions 36 while ensuring the productivity.

In order to obtain the environment having a relative humidity of 1 to 59%, the liquid drug drying step is preferably performed in a thermostatic room or a thermostatic tank having a humidity adjustment function.

In the liquid drug drying step, the liquid drug 52 is dried to be solidified so that the liquid drug 52 is reduced in size compared to the size when the filling with the liquid drug 52 is performed. Accordingly, in the releasing step, the first layers 22 can be easily released off from the needle-like recessed portions 36 of the mold 34.

### (Base-material liquid filling step)

Next, as illustrated in Fig. 23C, the mesh structure body 16 having an area which covers, in a plan view, at least the area of the needle-like recessed portions 36 is placed on the mold 34.

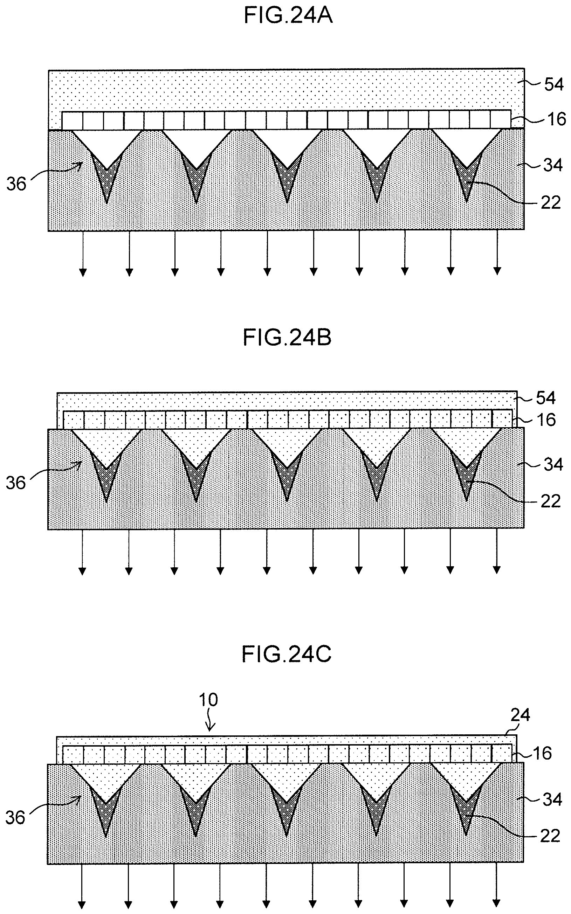

Next, as illustrated in Fig. 24A, a base material liquid 54 which is a polymer solution not containing a drug is applied to the mesh structure body 16, with a dispenser, from a side opposite to the mold 34. An amount of the base material liquid 54 exceeding spaces in the needle-like recessed portions 36 is applied. Instead of the coating using a dispenser, bar coating, spin coating, coating using, e.g., a spray, etc. can be adopted.

Next, as illustrated in Fig. 24B, the base material liquid 54 applied onto the mesh structure body 16 reaches the inside of the needle-like recessed portions 36 due to the gravity. Accordingly, the needle-like recessed portions 36 can be filled with the base material liquid 54. In the present embodiment, since the first layers 22 containing drugs are dried and solidified, the drugs contained in the first layers 22 can be prevented from being diffused to the base material liquid 54.

The mesh structure body 16 holds the base material liquid 54 with a capillary pressure and the base material liquid 54 diffuses in the meshes (through holes). As a result, for example, a problem of uneven application of the base material liquid 54, which arises because the base material liquid 54 is repelled on the mold 34 when the water-repellent mold 34 is used, can be solved. Moreover, even if the base material liquid 54 is applied to protrude from the mesh structure body 16, the base material liquid 54 is repelled and moved to be concentrated into the mesh structure body 16, and thus, the coating of the base material liquid 54 can be forcibly shaped into the shape of the mesh structure body 16. In order to prevent bubbles from being mixed when the base material liquid 54 is filled, it is preferable that a material having gas permeability is used for the mold 34 and the filling of the base material liquid 54 is performed while sucking on the mold 34 from a side opposite to the side which is filled with the base material liquid 54.

The mesh structure body 16 may be set after the base material liquid 54 is filled. However, depending on the physical properties of the base material liquid 54 or the design of the mesh structure body 16, the mesh structure body 16 may float on the base material liquid 54 due to effects of the specific gravity, the viscosity, the surface tension, etc. When the mesh structure body 16 floats, the anchor effect of the base material liquid 54 on the mesh structure body 16 is weakened, and thus, the mesh structure body 16 may be separated from the sheet portion. This problem can be avoided by placing the mesh structure body 16 on the mold 34, and filling the needle-like recessed portions 36 with the base material liquid 54 via the mesh structure body 16.

In the present embodiment, after the first layers 22 are formed, as described above, the base material liquid 54 is put on the first layers 22 within the needle-like recessed portions 36. When the mold has gas permeability, performing sucking from the rear surface of the mold 34 causes bubbles (air) remaining in the needle-like recessed portions 36 to pass through the inside of the mold 34 so that the bubbles disappear.

As a result of adjusting the amount of the base material liquid 54 to be put in the needle-like recessed portions 36 of the mold 34, the transdermal absorption sheet 10 in which the mesh structure body 16 is embedded in the sheet portion 14, and the transdermal absorption sheet 10 in which a part of the mesh structure body 16 is embedded in the sheet portion 14 and the other part is exposed from the sheet portion 14 can be manufactured.

### (Base-material liquid drying step)

Next, as illustrated in Fig. 24C, the base material liquid 54 is dried and solidified to form the second layers 24 not containing a drug on the first layers 22 containing drugs. The transdermal absorption sheet 10 having the first layers 22, the second layers 24, and the mesh structure body 16 is manufactured.

In the base-material liquid drying step, in order to obtain an environment having a desired temperature and humidity, it is preferable that the base-material liquid drying step is performed in a thermostatic room or a thermostatic tank having a temperature-humidity adjustment function, for example. Alternatively, the temperature may be controlled locally by using a hot plate or a cool plate and placing the mold on the plate. The temperature and humidity condition for drying is adjusted as appropriate depending on the physical properties of the base material liquid 54. However, it is preferable that the temperature is higher than 1°C but not higher than 45°C and the relative humidity is 40 to 80%RH. In addition, it is preferable that the air velocity in performing the drying is 0 to 5 m/s.

In the base-material liquid drying step, the base material liquid 54 is reduced in volume due to being dried. When the base material liquid 54 is in close contact with the mold 34 during drying, the volume is reduced in the film thickness direction of the sheet portion, thereby reducing the film thickness. In the present embodiment, since the mesh structure body 16 is included in the sheet portion 14, the mesh structure body 16 can suppress the sheet portion 14 from being curled in drying. Since the sheet portion 14 can be suppressed from being curled, breakage or bending of the needle-like protruding portions of the transdermal absorption sheet 10 can be suppressed.

### (Releasing step)

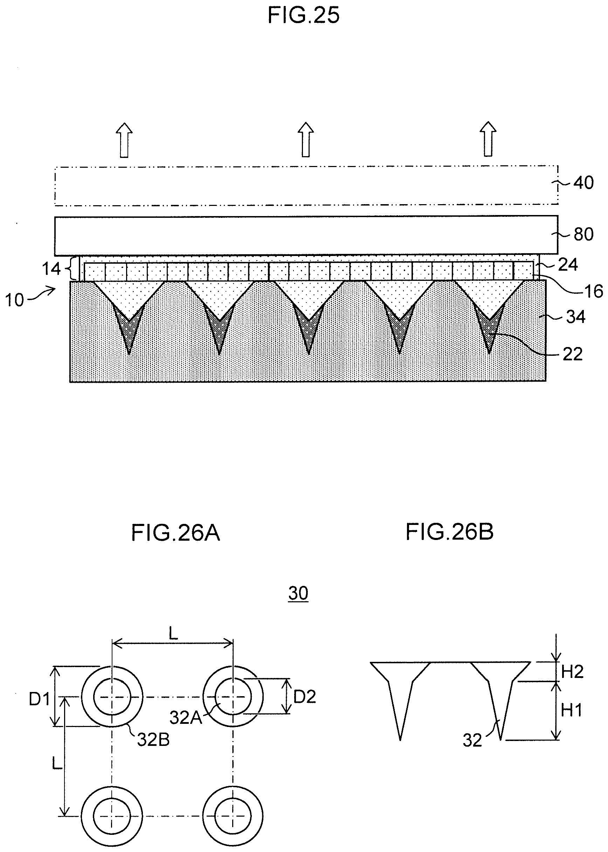

There is no limitation on a method of releasing the transdermal absorption sheet 10 from the mold 34. The needle-like protruding portions are demanded not to be bended or broken when being released off. Specifically, as illustrated in Fig. 25, a method can be adopted of attaching a sheet-like base material 80 having an adhesive layer onto the second main surface 14B of the sheet portion 14 of the transdermal absorption sheet 10, providing a sucker (not illustrated) on the rear surface of the base material 80, and pulling the base material 80 vertically upward while sucking the base material 80 with air. The transdermal absorption sheet 10 is released off from the mold 34, and thus, the transdermal absorption sheet 10 is completed.

When a structure with needle-like protruding portions having a high aspect ratio is released off from the mold 34, as in the present embodiment, a strong stress is usually applied because the contact area is large. When microneedles, which are the needle-like protruding portions, are broken, the microneedles remain in the needle-like recessed portions 36 without being removed from the mold 34. Accordingly, the transdermal absorption sheet 10 having a defect is manufactured. In the present embodiment, it is preferable that the mold 34 is made of a material which is very easy to be released off. In addition, as a result of forming the mold 34 of a soft material having a high elasticity, a stress to be applied to the microneedles in releasing can be relaxed.

In the present embodiment, the method of manufacturing the transdermal absorption sheet 10 by forming the first layers 22 and the second layers 24 of the liquid drug 52 and the base material liquid 54 has been described. However, the present disclosure is not limited to this method. For example, the mesh structure body 16 having an area including, in a plan view, at least the area of the needle-like recessed portions 36 is set on the mold 34 which has two-dimensionally arranged needle-like recessed portions, the needle-like recessed portions 36 are filled with a polymer solution via the mesh structure body 16, the polymer solution filled in the needle-like recessed portions 36 is dried, and then, the dried polymer solution and the mesh structure body 16 are released off from the mold 34. Thus, the transdermal absorption sheet 10 can be manufactured.

### (Final drying step)

The released sheet is left to stand in an environment having a predetermined temperature and a predetermined humidity, and is dried until a desired water content value of the entire sheet is obtained. It is preferable that the water content, which has an influence on the stability of drugs, is set as low as possible. The recommended water content is 5% or less by weight. However, depending on the properties of the drug and the base material, the water content may be approximately 10%. In the final drying step, drying is performed in an environment having a temperature and a humidity at which the equilibrium water content is equal to a desired water content. Drying may be performed in a decompression environment, as needed. When the desired water content is obtained in the base-material drying step, the final drying step can be omitted.

### (Degassing step)

It is preferable that the liquid drug 52 and/or the base material liquid 54 is degassed before the liquid drug filling step and/or the base-material liquid filling step. Through the degassing, bubbles contained in the liquid drug 52 and the base material liquid 54 can be removed before filling the needle-like recessed portions 36 of the mold 34. For example, in the degassing step, bubbles each having a diameter of 100 µm to several mm are removed. Air bubbles can be facilitated to be dissolved in the polymer solution by degassing at least one of the liquid drug 52 and the base material liquid 54.

Examples of the degassing method include (1) a method of leaving the liquid drug 52 in a decompression environment for 1 to 15 minutes, (2) a method of vibrating a container storing the liquid drug 52 with ultrasonic waves for 5 to 10 minutes, (3) a method of applying ultrasonic waves to the liquid drug 52 in a decompression environment, and (4) a method of replacing dissolved gas with helium by sending helium gas into the liquid drug 52. The degassing methods (1) to (4) also can be used for the base material liquid 54.

### Examples

The present disclosure is further specifically described using examples. Thus, the scope of the present invention should not be interpreted in a limited manner based on the specific examples described below.

### <Example 1>

### (Production of mold)

The original plate 30 was produced by grinding a surface of a smooth Ni plate having a side of 40 mm so as to form the needle-like protruding portions 32 which were arranged at a pitch L of 1000 µm in two-dimensional array with 10 columns and 10 rows and which each had a needle-like structure in which a cone 32A was formed on a truncated cone 32B, as illustrated in Figs. 26A and 26B. The cone 32A had a diameter D2 of 300 µm and a height H1 of 500 µm. The truncated cone 32B had a bottom face with a diameter D1 of 500 µm and a height H2 of 150 µm. A film having a thickness of 0.6 mm was formed on the original plate 30 by using silicone rubber (SILASTIC-MDX4-4210 manufactured by Dow Corning Toray Co., Ltd., SILASTIC is a registered trademark), was thermally cured with the tips of the cones on the original plate 30 protruding by 50 µm from the surface of the film, and then, was released off. Thus, an inverted product made of silicone rubber and having through holes with a diameter of approximately 30 µm was produced. The inverted product made of silicone rubber was trimmed so as to leave a planar portion with a side of 30 mm, which has the two-dimensionally arranged 10 columns × 10 rows needle-like recessed portions formed on the central portion thereof. The portion thus obtained was used as a mold. One surface of the mold having the wider openings of needle-like protruding portions was used as the front surface of the mold, and the other surface of the mold having the through holes (air vent holes) with a diameter of 30 µm was used as the rear surface of the mold.

### (Preparation of polymer solution containing drug)

Hydroxyethyl starch (manufactured by Fresenius Kabi) was dissolved into water to prepare an 8% water solution. As a drug, 2% by mass of human serum albumin (manufactured by Wako Pure Chemical Industries, Ltd.) was added to the solution to obtain a liquid drug containing the drug. After being prepared, the liquid drug was left in a decompression environment having a pressure of 3 kPa for four minutes to degas the liquid drug.

### (Preparation of polymer solution not containing drug)

Hydroxypropyl cellulose (manufactured by Nippon Soda Co., Ltd.) was dissolved into water to prepare a 30% water solution. The prepared solution was used as a polymer solution not containing a drug, that is, a base material liquid. After being prepared, the solution was left in a decompression environment having a pressure of 3 kPa for four minutes to degas the solution.

### (Liquid drug filling step, liquid drug drying step)

A liquid drug filling apparatus includes: a driving unit having an X-axis drying unit for controlling the relative positional coordinates of the mold and the nozzle and a Z-axis driving unit; a liquid supply apparatus (Nano master SMP-III manufactured by Musashi Engineering, Inc.) to which the nozzle can be attached; a suction platform for fixing the mold, a laser displacement sensor (HL-C201A manufactured by Panasonic Corporation) for measuring the surface shape of the mold; load cells (LCX-A-500N manufactured by Kyowa Electronic Instruments Co., Ltd.) for measuring the nozzle pressuring force, and a control system for controlling the Z-axis on the basis of measurement value data of the surface shape and the pressuring force.

A gas permeable film (Poreflon FP-010 manufactured by Sumitomo Electric Industries, Ltd.) having one side of 15 mm was placed on the horizontal suction platform, and the mold was placed on the film such that the front surface of the mold is the upper side. Decompression was performed from the rear surface of the mold at a gauge pressure of 90 kPa as a suction pressure, thereby fixing the gas permeable film and the mold to the vacuum platform.

A stainless nozzle having the shape illustrated in Fig. 17 was prepared, and a slit-like opening having a length of 12 mm and a width of 0.2 mm was formed at the center of the lip portion having a length of 20 mm and a width of 2 mm. The nozzle was connected to a liquid drug tank. A solution containing 3 mL of a drug was put into the liquid drug tank and the inside of the nozzle. The nozzle was adjusted such that the opening was parallel with the first line constituted of a plurality of needle-like recessed portions formed on the front surface of the mold. The nozzle was adjusted to the position away, in a direction opposite to the second line, from the first line by 2 mm, and pressed against the mold with a pressure (a pressuring force) of 0.14 kgf/cm² (1.4 N/cm²). The liquid supply apparatus discharged the solution containing the drug from the opening at 0.31 µL/sec for ten seconds, while the nozzle was pressed, the Z-axis was controlled such that variation in pressuring force was kept within ±0.05 kgf/cm² (0.49 N/cm²), and the nozzle was being moved at 1 mm/sec in a direction orthogonal to the longitudinal direction of the opening. At the position away, in the direction opposite to the ninth line of the two-dimensionally arranged needle-like recessed portions, from the tenth line by 2 mm, the movement of the nozzle was stopped, and the nozzle was separated from the mold.

The mold was filled with the liquid drug in the environment having a temperature of 5°C and a relative humidity of 50%RH, and was left to stand for 30 minutes. Thus, the liquid drug was localized at the tips of the needle-like recessed portions.

### (Base-material liquid filling step, base-material liquid drying step)

The mold filled with the liquid drug was sucked and fixed to the suction apparatus. A mesh structure body (having 100 meshes per one inch and a wire diameter of 0.1 mm) made of SUS 316 and processed to have a round shape with a diameter of 20 mm was placed at the center of the region of the needle-like recessed portions of the mold. Autoclave sterilization had been performed on the mesh structure body and it had been confirmed through a microscope that no foreign substance had been attached to the mesh structure body before use. While sucking was being performed on the rear surface of the mold, approximately 200 mg of the base-material liquid was applied onto the mesh structure body. The base-material liquid which was applied to protrude from the mesh structure body gathered at the center of the mesh structure body by being repelled from the mold surface. The base-material liquid passed through the mesh structure body due to the gravity and filled the needle-like recessed portions of the mold. The process proceeded to the base-material liquid drying step in a state where the mesh structure body was embedded in the base material liquid.

The mold was placed on a 35°C hot plate in an environment having a temperature of 23°C, a relative humidity of 45%RH, and an air velocity of 0.4 m/s, and was left to stand for six hours to be dried. The water contents reached to 5% or less.

### (Releasing step)

The transdermal absorption sheet was released off from the mold by a method of pulling the transdermal absorption sheet upward while sucking the sheet with air. The transdermal absorption sheet containing human serum albumin eccentrically located at the tip was formed. The transdermal absorption sheet includes: the sheet portion including the layer not containing a drug and the mesh structure body; and the needle-like protruding portions having a three-dimensionally arranged structure formed of the first layers containing the drugs and the second layers not containing a drug placed on the sheet portion. The sheet portion and the second layer not containing a drug were formed of the same material.

### (Final drying step)

Since the water content reached 5% or less in the base-material liquid drying step, the final drying step was omitted.

### (Shape of transdermal absorption sheet)

The mesh structure body functioned as a reinforcing material. As a result, no curl was generated after releasing. After releasing, the transdermal absorption sheet was heated on a 105°C hot plate for an hour but no change occurred in shape of the sheet portion. The transdermal absorption sheet was exposed to an environment having a temperature of 35°C and a humidity of 80%RH for an hour, but no change occurred in shape of the sheet portion. The mesh structure body succeeded in suppressing the shape change caused by drying and moisture absorption. To check the impact resistance at a time of puncture, the transdermal absorption sheet was nailed onto a silicone rubber sheet (Shore A hardness of 50) having a thickness of 5 mm, at a speed of 8 m/s. The sheet portion was not broken because no breakage or crack was generated.

### <Comparative Example 1>

The transdermal absorption sheet was manufactured without using the mesh structure body. When the base material liquid was poured, a stainless thin plate having an opening with a diameter of 24 mm and having a thickness of 300 µm was prepared as a mold. The mold was filled with the liquid drug, and was sucked and fixed to the suction device. The region of the needle-like recessed portions of the mold was positioned so as to be included in the opening of the stainless thin plate, and the stainless thin plate was placed over the front surface of the mold. The base material liquid was poured into the opening of the stainless thin plate, and the excess base material liquid was removed by a squeegee or a round bar. The inside of the opening was filled with approximately 200 mg of the base material liquid. Except for this, the same operations as those in the example were performed.

No mesh structure body was used, but 6 hours were required for drying. After being released off, the transdermal absorption sheet was placed on a flat place such that the needle-like protruding portions face upward. A shape having a curl, with the end portions off the surface by approximately 0.5 to 1 mm was formed.

The impact resistance was checked in the same way as that in the example. The sheet portion was broken in some cases because a breakage or a crack was generated.

### <Comparative Example 2>

A porous structure body obtained by sintering metal particles was used instead of the mesh structure body. The porous structure body used was made of stainless, had a circular shape with a diameter of 20 mm, a thickness of 1 mm, and had a porosity of 36 to 48%. Autoclave sterilization had been performed on the porous structure body and it had been confirmed through a microscope that no foreign substance had been attached to the mesh structure body before use. However, at the inner part of the porous structure body, confirming whether sterilization had been completed and whether no foreign substance had been attached was failed.

Approximately 200 mg of the base material liquid was dripped onto the center of the needle-like recessed portion region of the mold, and the porous structure body was set on the base material liquid to spread the base material liquid. Except for this, the same operations as those in the example were performed.

In a case where the porous structure body was used, the center portion thereof was still wet even after being dried for six hours. Thus, no needle-like protruding portions were formed.

## Claims

1. A method of manufacturing a transdermal absorption sheet (10), the method comprising, in this order:
a filling step of placing, on a mold (34) having two-dimensionally arranged needle-like recessed portions (36), a mesh structure body (16) which has an area, in a plan view, covering at least a region of the needle-like recessed portions (36), and filling the needle-like recessed portions (36) with a polymer solution via the mesh structure body (16);
a drying step of drying the polymer solution filled in the needle-like recessed portions (36); and
a releasing step of releasing the dried polymer solution and the mesh structure body (16) from the mold (34).

2. The method of manufacturing a transdermal absorption sheet (10) according to claim 1, wherein
the filling step includes:
filling the needle-like recessed portions (36) of the mold (34) with a liquid drug which is a polymer solution containing a drug before the mesh structure body (16) is placed;
drying the liquid drug filled in the needle-like recessed portions (36) to form a drug layer (22) containing the drug; and
filling the needle-like recessed portions (36) of the mold (34) on the drug layer (22) via the mesh structure body (16) with a base material liquid which is a polymer solution not containing a drug, after the mesh structure body (16) is placed.

3. The method of manufacturing a transdermal absorption sheet (10) according to claim 1 or 2, wherein the mesh structure body (16) is made of metal.

4. The method of manufacturing a transdermal absorption sheet (10) according to claim 3, wherein the metal is stainless steel.

5. The method of manufacturing a transdermal absorption sheet (10) according to any one of claims 1 to 4, wherein
by drying the polymer solution in the drying step, the mesh structure body (16) is embedded in the dried polymer solution.

6. The method of manufacturing a transdermal absorption sheet (10) according to any one of claims 1 to 4, wherein
by drying the polymer solution in the drying step, the mesh structure body (16) has a portion embedded in the dried polymer solution and a portion exposed from the dried polymer solution.

7. The method of manufacturing a transdermal absorption sheet (10) according to any one of claims 1 to 6, wherein the mesh structure body (16) is a woven net.

8. The method of manufacturing a transdermal absorption sheet (10) according to claim 7, wherein a number of meshes of the mesh structure body (16) is within a range of 12 to 635 meshes, and a wire diameter of the mesh structure body (16) is within a range of 0.02 to 1.0 mm.

9. The method of manufacturing a transdermal absorption sheet (10) according to claim 2, wherein the drug is at least one of peptide, protein, nucleic acid, polysaccharide, a vaccine, a medical compound, and a cosmetic component.

10. The method of manufacturing a transdermal absorption sheet (10) according to any one of claims 1 to 9, wherein
in the filling step, the polymer solution is filled in the needle-like recessed portions (36) by gravity.

11. The method of manufacturing a transdermal absorption sheet (10) according to any one of claims 1 to 10, wherein
in the filling step, the mesh structure body (16) holds the polymer solution with a capillary pressure.

## Patentansprüche

1. Verfahren zum Fertigen eines transdermalen Absorptionsflachstücks (10), umfassend in der genannten Reihenfolge:
einen Befüllschritt, bei dem auf einer Form (34) mit zweidimensional angeordneten, nadelähnlichen Vertiefungen (36) ein Maschenstrukturkörper (16) platziert wird, der in einer Draufsicht eine Fläche aufweist, die mindestens eine Zone der nadelähnlichen Vertiefungen (36) abdeckt, und die nadelähnlichen Vertiefungen (36) über den Maschenstrukturkörper (16) mit einer Polymerlösung befüllt werden;
einen Trocknungsschritt des Trocknens der in die nadelähnlichen Vertiefungen (36) gefüllten Polymerlösung; und
einen Ablöseschritt des Ablösens der getrockneten Polymerlösung und des Maschenstrukturkörpers (16) von der Form (36).

2. Verfahren nach Anspruch 1, bei dem
der Befüllschritt enthält:
Befüllen der nadelähnlichen Vertiefungen (36) der Form (34) mit einem Flüssigmedikament, das ist eine Polymerlösung, die ein Medikament enthält, bevor der Maschenstrukturkörper (16) platziert wird;
Trocknen des in die nadelähnlichen Vertiefungen (36) gefüllten Flüssigmedikaments, um eine Medikamentenschicht (22) zu bilden, welches das Medikament enthält; und
Befüllen der nadelähnlichen Vertiefungen (36) der Form (34) auf der Medikamentenschicht (22) über den Maschenstrukturkörper (16) mit einer Basismaterialflüssigkeit, das ist eine Polymerlösung, die kein Medikament enthält, nachdem der Maschenstrukturkörper (16) platziert wurde.

3. Verfahren nach Anspruch 1 oder 2, bei dem der Maschenstrukturkörper (16) aus Metall besteht.

4. Verfahren nach Anspruch 3, bei dem das Metall Edelstahl ist.

5. Verfahren nach einem der Ansprüche 1 bis 4, bei dem
durch Trocknen der Polymerlösung in dem Trocknungsschritt der Maschenstrukturkörper (16) in die getrocknete Polymerlösung eingebettet wird.

6. Verfahren nach einem der Ansprüche 1 bis 4, bei dem
durch Trocknen der Polymerlösung in dem Trocknungsschritt der Maschenstrukturkörper (16) mit einem Teil in die getrocknete Polymerlösung eingebettet wird und mit einem Teil aus der getrockneten Polymerlösung freiliegt.

7. Verfahren nach einem der Ansprüche 1 bis 6, bei dem der Maschenstrukturkörper (16) ein Webnetz ist.

8. Verfahren nach Anspruch 7, bei dem eine Anzahl von Maschen des Maschenstrukturkörpers (16) in einem Bereich von 12 bis 635 Maschen liegt, und ein Drahtdurchmesser des Maschenstrukturkörpers (16) in einem Bereich von 0,02 bis 1,0 mm liegt.

9. Verfahren nach Anspruch 2, bei dem Medikament ein Peptid, ein Protein, Nucleinsäure, Polysaccharid, ein Impfstoff, eine medizinische Verbindung und/oder eine kosmetische Komponente ist.

10. Verfahren nach einem der Ansprüche 1 bis 9, bei dem
in dem Befüllschritt die Polymerlösung in die nadelähnlichen Vertiefungen (36) durch Schwerkraft gefüllt wird.

11. Verfahren nach einem der Ansprüche 1 bis 10, bei dem
in dem der Befüllschritt der Maschenstrukturkörper (16) die Polymerlösung durch Kapillardruck hält.

## Revendications

1. Procédé de fabrication d'une feuille d'absorption transdermique (10), comprenant les étapes suivantes, dans cet ordre :
une étape de remplissage pour placer, sur un moule (34) présentant des portions en retrait en forme d'aiguilles (36) agencées de manière bidimensionnelle, un corps à structure maillée (16), lequel présente une zone, en vue en plan, couvrant au moins une région des portions en retrait en forme d'aiguilles (36), et remplir les portions en retrait similaires à des aiguilles (36) avec une solution polymère via le corps à structure maillée (16) ;
une étape de séchage pour sécher la solution polymère remplissant les portions en retrait en forme d'aiguilles (36), et
une étape de démoulage pour démouler la solution polymère séchée et le corps à structure maillée (16) du moule (34).

2. Procédé de fabrication d'une feuille d'absorption transdermique (10) selon la revendication 1, dans lequel
l'étape de remplissage inclut les étapes consistant à :
remplir les portions en retrait en forme d'aiguilles (36) du moule (34) avec un médicament liquide, lequel est une solution polymère contenant un médicament avant placement du corps à structure maillée (16) ;
sécher le médicament liquide remplissant les portions en retrait en forme d'aiguilles (36) pour former une couche médicamenteuse (22) contenant le médicament, et
remplir les portions en retrait en forme d'aiguilles (36) du moule (34) sur la couche médicamenteuse (22) via le corps à structure maillée (16) avec un liquide de matière de base, lequel est une solution polymère ne contenant pas de médicament, après placement du corps à structure maillée (16).

3. Procédé de fabrication d'une feuille d'absorption transdermique (10) selon la revendication 1 ou 2, dans lequel le corps à structure maillée (16) est en métal.

4. Procédé de fabrication d'une feuille d'absorption transdermique (10) selon la revendication 3, dans lequel le métal est de l'acier inoxydable.

5. Procédé de fabrication d'une feuille d'absorption transdermique (10) selon l'une quelconque des revendications 1 à 4, dans lequel
par le séchage de la solution polymère lors de l'étape de séchage, le corps à structure maillée (16) est intégré dans la solution polymère séchée.

6. Procédé de fabrication d'une feuille d'absorption transdermique (10) selon l'une quelconque des revendications 1 à 4, dans lequel
par le séchage de la solution polymère lors de l'étape de séchage, le corps à structure maillée (16) présente une portion intégrée dans la solution polymère séchée et une portion exposée à partir de la solution polymère séchée.

7. Procédé de fabrication d'une feuille d'absorption transdermique (10) selon l'une quelconque des revendications 1 à 6, dans lequel le corps à structure maillée (16) est un filet tissé.

8. Procédé de fabrication d'une feuille d'absorption transdermique (10) selon la revendication 7, dans lequel un nombre de mailles du corps à structure maillée (16) est compris dans les limites d'une plage de 12 à 635 mailles, et un diamètre de fil du corps à structure maillée (16) est compris dans les limites d'une plage de 0,02 à 1,0 mm.

9. Procédé de fabrication d'une feuille d'absorption transdermique (10) selon la revendication 2, dans lequel le médicament est au moins un élément parmi un peptide, une protéine, un acide nucléique, un polysaccharide, un vaccin, un composé médical, et un composant de cosmétique.

10. Procédé de fabrication d'une feuille d'absorption transdermique (10) selon l'une quelconque des revendications 1 à 9, dans lequel
lors de l'étape de remplissage, la solution polymère séchée est remplie dans les portions en retrait en forme d'aiguilles (36) par gravité.

11. Procédé de fabrication d'une feuille d'absorption transdermique (10) selon l'une quelconque des revendications 1 à 10, dans lequel
lors de l'étape de remplissage, le corps à structure maillée (16) contient la solution polymère avec une pression capillaire.
